# NEW EUROPEAN PATENT SPECIFICATION

(11) **EP 3 145 349 B2**
(45) Date of publication and mention of the opposition decision: **12.10.2022**
(45) Mention of the grant of the patent: 03.07.2019
(21) Application number: 15727771.6
(22) Date of filing: 19.05.2015
(51) Int. Cl.: A24F 47/00

(54) **ELECTRICALLY-POWERED AEROSOL DELIVERY SYSTEM**
ELEKTRISCH BETRIEBENES AEROSOLABGABESYSTEM
SYSTÈME D'ADMINISTRATION D'AÉROSOL À ALIMENTATION ÉLECTRIQUE

(30) Priority: 20.05.2014 US 201414282768
(43) Date of publication of application: 29.03.2017
(60) Divisional application: 19162965.8
(73) Proprietor: RAI Strategic Holdings, Inc., Winston-Salem, NC 27101 (US)
(72) Inventor: SEARS, Stephen Benson, Siler City, North Carolina 27344 (US); TALUSKIE, Karen V., Winston-Salem, North Carolina 27104 (US); DAVIS, Michael F., Clemmons, North Carolina 27012 (US); ADEME, Balager, Winston-Salem, North Carolina 27127 (US); DUGGINS, Donna Walker, Winston-Salem, North Carolina 27107 (US); GERARDI, Anthony Richard, Winston-Salem, North Carolina 27104 (US)
(74) Representative: D Young & Co LLP
(86) International application number: PCT/US2015/031563
(87) International publication number: WO 2015/179388

(56) References cited:
- WO-A1-2013/116558
- WO-A1-2013/120855
- US-A1- 2011 226 236
- US-A1- 2012 204 889
- US-B2- 7 726 320

## Description

### BACKGROUND

### Field of the Disclosure

The present disclosure relates to aerosol delivery devices and systems, such as smoking articles; and more particularly, to aerosol delivery devices and systems that utilize electrically-generated heat for the production of aerosol (e.g., smoking articles commonly referred to as electronic cigarettes). The aerosol delivery devices and systems may be configured to heat an aerosol precursor, which incorporates materials that may be, though not necessarily, made or derived from tobacco or otherwise incorporate tobacco, and which are capable of vaporizing to form an inhalable aerosol for human consumption.

### Description of Related Art

Many smoking devices have been proposed through the years as improvements upon, or alternatives to, smoking products that require combusting tobacco for use. Many of those devices purportedly have been designed to provide the sensations associated with cigarette, cigar, or pipe smoking, but without delivering considerable quantities of incomplete combustion and pyrolysis products that result from the burning of tobacco. To this end, there have been proposed numerous smoking products, flavor generators, and medicinal inhalers that utilize electrical energy to vaporize or heat a volatile material, or attempt to provide the sensations of cigarette, cigar, or pipe smoking without burning tobacco to a significant degree. See, for example, the various alternative smoking articles, aerosol delivery devices and heat generating sources set forth in the background art described in U.S. Pat. App. Pub. Nos. 2013/0255702 to Griffith, Jr. et al.; and 2014/0096781 to Sears et al.. U.S. Pat. No. 7,726,320,Robinson et al. discloses a smoking article that possesses at least one form of tobacco and a mouth end piece that is used by the smoker to inhale components of the tobacco that are generated by the action of heat upon components of the tobacco.

It would be desirable to provide an electrically-powered aerosol delivery system that is capable of allowing the user thereof to draw aerosol that is highly flavorful. It may also be desirable for the aerosol to be provided under pleasing or comfortable conditions upon being drawn into the mouth of the user.

### SUMMARY OF THE DISCLOSURE

The present disclosure relates to aerosol delivery systems. Such systems have the ability to generate aerosol as a result of heat generated by electrical power sources, and to deliver aerosol that is intended to be drawn into the mouth of a user. Of particular interest are aerosol delivery systems that provide components of tobacco in an aerosol form, such as is provided to smokers by devices commonly known or characterized as electronic cigarettes. As used herein, the term "aerosol" is meant to include vapors, gases, aerosols, and/or particulate matter of a form or type suitable for human inhalation, whether visible or not, and whether or not of a form that might be considered to be "smoke-like."

The above and other needs are met by aspects of the present disclosure which, in one aspect, provides an aerosol delivery system as defined in claim 1, and a method of forming an aerosol delivery system as defined in claim 19.

Such an aerosol delivery system may comprise a control body portion, wherein the control body portion includes a first elongate tubular member having opposed ends, and a power source disposed therein. A cartridge body portion includes a second tubular member having opposed first and second ends. One of the first and second ends of the cartridge
body portion is removably engaged with one of the opposed ends of the control body portion. The cartridge body portion further comprises a first aerosol generation arrangement disposed within the second tubular member, and configured to operably engage the power source upon engagement between the one of the opposed ends of the control body portion and the one of the first and second ends of the cartridge body portion. The other of the first and second ends of the cartridge body
portion is further configured as a mouth-engaging end. The cartridge body portion further includes a second aerosol generation arrangement within the second tubular member disposed between the first aerosol generation arrangement and the mouth-engaging end. In some aspects, the second aerosol generation arrangement may further include one or more aerosol generation elements, wherein the one or more (at least one) aerosol generation elements may be selected from the group
consisting of granules, pellets, beads, discrete small units, carbon pieces, extruded carbon pieces, ceramic beads, marumarized tobacco pieces, extruded or compressed cylindrical or spherical elements, milled tobacco lamina, fillers, flavors, visible aerosol forming materials, binders, ovoid elements, irregularly shaped elements, shredded pieces, flakes, elements including tobacco, elements including a visible aerosol-forming material, adsorbent objects, absorbent objects,
capsules, microcapsules, a honeycomb monolith, a single porous structure, and combinations thereof.

Another aspect of the present disclosure provides a method of forming an aerosol delivery system. Such a method may comprise removably engaging one end of a first elongate tubular member with a first end of a second tubular member, wherein the first elongate tubular member is configured as a control body portion and having a power source disposed therein, and the second tubular member is configured as a cartridge body portion and having a first aerosol generation arrangement disposed therein. The first aerosol generation arrangement is configured to operably engage the power source upon engagement between the one end of the control body portion and the first end of the cartridge body portion. The method may also comprise inserting a second aerosol generation arrangement within the second tubular member of the cartridge body portion, between the first aerosol generation arrangement and a second end of the second tubular member, wherein the second end is opposed to the first end and is configured as a mouth-engaging end. In some instances, inserting the second aerosol generation arrangement within the second tubular member may further comprise inserting one or more aerosol generation elements, at least partially forming the second aerosol generation arrangement, into the second tubular member, wherein the one or more (at least one) aerosol generation elements is selected from the group consisting of granules, pellets, beads, discrete small units, carbon pieces, extruded carbon pieces, ceramic beads, marumarized tobacco pieces, extruded or compressed cylindrical or spherical elements, milled tobacco lamina, fillers, flavors, visible aerosol forming materials, binders, ovoid elements, irregularly shaped elements, shredded pieces, flakes, elements including tobacco, elements including a visible aerosol-forming material, adsorbent objects, absorbent objects, capsules, microcapsules, a honeycomb monolith, a single porous structure, and combinations thereof.

The present disclosure thus includes, without limitation, the following embodiments:
Embodiment 1: An aerosol delivery system, comprising a control body portion including a first elongate tubular member having opposed ends, and a power source disposed therein; and a cartridge body portion including a second tubular member having opposed first and second ends, wherein one of the first and second ends is removably engaged with one of the opposed ends of the control body portion, wherein the cartridge body portion further comprises a first aerosol generation arrangement disposed within the second tubular member and configured to operably engage the power source upon engagement between the one of the opposed ends of the control body portion and the one of the first and second ends of the cartridge body portion, wherein the other of the first and second ends of the cartridge body portion is optionally further configured as a mouth-engaging end, and wherein the cartridge body portion further includes a second aerosol generation arrangement within the second tubular member disposed between the first aerosol generation arrangement and the mouth-engaging end.
Embodiment 2: The aerosol delivery system of any preceding or subsequent embodiment, or combinations thereof, wherein the second aerosol generation arrangement further includes at least one aerosol-generating element.
Embodiment 3: The aerosol delivery system of any preceding or subsequent embodiment, or combinations thereof, wherein the at least one aerosol-generating element is selected from the group consisting of granules, pellets, beads, discrete small units, carbon pieces, extruded carbon pieces, ceramic beads, marumarized tobacco pieces, extruded or compressed cylindrical or spherical elements, milled tobacco lamina, fillers, flavors, visible aerosol forming materials, binders, ovoid elements, irregularly shaped elements, shredded pieces, flakes, elements including tobacco, elements including a visible aerosol-forming material, adsorbent objects, absorbent objects, capsules, microcapsules, a honeycomb monolith, a single porous structure, and combinations thereof.
Embodiment 4: The aerosol delivery system of any preceding or subsequent embodiment, or combinations thereof, further comprising a first separating element disposed within the second tubular member between the first aerosol generation arrangement and the second aerosol generation arrangement, the first separating element being one of heat-conductive and air permeable.
Embodiment 5: The aerosol delivery system of any preceding or subsequent embodiment, or combinations thereof, wherein the first separating element extends along a longitudinal axis between opposed ends so as to define a thickness, the thickness of the first separating element being configured to space the second aerosol generation arrangement from a heating element of the first aerosol generation arrangement.
Embodiment 6: The aerosol delivery system of any preceding or subsequent embodiment, or combinations thereof, further comprising a second separating element disposed within the second tubular member between the second aerosol generation arrangement and the mouth-engaging end, the second separating element being one of heat-conductive and air permeable.
Embodiment 7: The aerosol delivery system of any preceding or subsequent embodiment, or combinations thereof, wherein the second aerosol generation arrangement comprises a cartridge having an elongate tubular body and opposed end members, each of the end members being one of heat-conductive and air permeable, the elongate tubular body being further configured to receive at least one aerosol-generating element and to cooperate with the opposed end members to contain the at least one aerosol-generating element therein, the cartridge being configured to be received by the second tubular member.
Embodiment 8: The aerosol delivery system of any preceding or subsequent embodiment, or combinations thereof, wherein the first aerosol generation arrangement comprises a liquid reservoir disposed within the second tubular member and configured to receive an aerosol precursor substance used by the first aerosol generation arrangement to generate a first aerosol. Embodiment 9: The aerosol delivery system of any preceding or subsequent embodiment, or combinations thereof, wherein the aerosol precursor substance is one of flavorant-free and acid-free.
Embodiment 10: The aerosol delivery system of any preceding or subsequent embodiment, or combinations thereof, wherein the aerosol precursor substance is one of glycerin, propylene glycol, water, saline, nicotine, and combinations thereof.
Embodiment 11: The aerosol delivery system of any preceding or subsequent embodiment, or combinations thereof, wherein the first aerosol generation arrangement includes a heating element configured to provide heat for producing a first aerosol, and the second aerosol generation arrangement includes at least one aerosol-generating element, the at least one aerosol-generating element being arranged to interact with the heat and the first aerosol, drawn therethrough toward the mouth-engaging end, in response to a suction applied to the mouth-engaging end of the cartridge body portion.
Embodiment 12: The aerosol delivery system of any preceding or subsequent embodiment, or combinations thereof, wherein the at least one aerosol-generating element of the second aerosol generation arrangement is configured to interact with one of the heat from the heating element of the first aerosol generation arrangement and the first aerosol generated by the first aerosol generation arrangement to produce a second aerosol.
Embodiment 13: The aerosol delivery system of any preceding or subsequent embodiment, or combinations thereof, wherein the first aerosol generated by the first aerosol generation arrangement is configured to interact with the second aerosol generated by the second aerosol generation arrangement to form a tertiary aerosol, drawn toward the mouth-engaging end in response to the suction applied thereto.
Embodiment 14: The aerosol delivery system of any preceding or subsequent embodiment, or combinations thereof, wherein the at least one aerosol-generating element of the second aerosol-generation arrangement is configured to interact with and impart an enhancement substance to the first aerosol generated by the first aerosol generation arrangement to produce an enhanced aerosol, drawn toward the mouth-engaging end in response to the suction applied thereto.
Embodiment 15: The aerosol delivery system of any preceding or subsequent embodiment, or combinations thereof, wherein the at least one aerosol-generating element of the second aerosol generation arrangement is configured to interact with and remove heat from the first aerosol generated by the first aerosol generation arrangement to produce a cooled aerosol, drawn toward the mouth-engaging end in response to the suction applied thereto.
Embodiment 16: An aerosol delivery system, comprising: a control body portion including a first elongate tubular member having opposed ends, and a power source disposed therein; a cartridge body portion including a second tubular member having opposed first and second ends, the first end being engaged with one of the opposed ends of the control body portion, the cartridge body portion further comprising a first aerosol generation arrangement disposed within the second tubular member and configured to operably engage the power source upon engagement between the one of the opposed ends of the control body portion and the first end of the cartridge body portion, the second end of the cartridge body portion facing toward a mouth-engaging end of the aerosol delivery system; and a second aerosol generation arrangement disposed between the first aerosol generation arrangement and the mouth-engaging end of the aerosol delivery system, the second aerosol generation arrangement being either removably engaged with the cartridge body portion or housed within the second tubular member of the cartridge body portion.
Embodiment 17: The aerosol delivery system of any preceding or subsequent embodiment, wherein the second aerosol generation arrangement further includes a plurality of aerosol-generating elements in the form of beads or pellets comprising at least one aerosol forming material.
Embodiment 18: The aerosol delivery system of any preceding or subsequent embodiment, wherein the aerosol-generating elements further comprise one or more of particulate tobacco, a tobacco extract, and nicotine, wherein the nicotine in free base form, salt form, as a complex, or as a solvate.
Embodiment 19: The aerosol delivery system of any preceding or subsequent embodiment, wherein the aerosol-generating elements further comprise one or more fillers, binders, flavorants, and combinations thereof.
Embodiment 20: The aerosol delivery system of any preceding or subsequent embodiment, wherein the aerosol-generating elements are smoke-treated.
Embodiment 21: The aerosol delivery system of any preceding or subsequent embodiment, wherein the second aerosol generation arrangement is housed within the second tubular member of the cartridge body portion and includes a plurality of aerosol-generating elements in the form of beads or pellets retained in place by a first air permeable separating element disposed within the second tubular member between the first aerosol generation arrangement and the second aerosol generation arrangement and a second separating element between the second aerosol generation arrangement and the mouth-engaging end.
Embodiment 22: The aerosol delivery system of any preceding or subsequent embodiment, wherein the second aerosol generation arrangement is removably engaged with the cartridge body portion and includes a plurality of aerosol-generating elements in the form of beads or pellets retained in place by a first air permeable separating element between the first aerosol generation arrangement and the second aerosol generation arrangement and a second separating element between the second aerosol generation arrangement and the mouth-engaging end.
Embodiment 23: A method of forming an aerosol delivery system, comprising removably engaging one end of a first elongate tubular member with a first end of a second tubular member, wherein the first elongate tubular member is configured as a control body portion and has a power source disposed therein, wherein the second tubular member is configured as a cartridge body portion and has a first aerosol generation arrangement disposed therein, and wherein the first aerosol generation arrangement is configured to operably engage the power source upon engagement between the one end of the control body portion and the first end of the cartridge body portion; and inserting a second aerosol generation arrangement within the second tubular member of the cartridge body portion, between the first aerosol generation arrangement and a second end of the second tubular member, wherein the second end is opposed to the first end and is configured as a mouth-engaging end.
Embodiment 24: The method of any preceding or subsequent embodiment, wherein said step of engaging a second aerosol generation arrangement with the cartridge body portion comprises inserting the second aerosol generation arrangement within the second tubular member of the cartridge body portion, between the first aerosol generation arrangement and a second end of the second tubular member, the second end being opposed to the first end and being configured as a mouth-engaging end.
Embodiment 25: The method of any preceding or subsequent embodiment, or combinations thereof, wherein inserting the second aerosol generation arrangement within the second tubular member further comprises inserting at least one aerosol-generating element, at least partially forming the second aerosol generation arrangement, into the second tubular member, the at least one aerosol-generating element being selected from the group consisting of granules, pellets, beads, discrete small units, carbon pieces, extruded carbon pieces, ceramic beads, marumarized tobacco pieces, extruded or compressed cylindrical or spherical elements, milled tobacco lamina, fillers, flavors, visible aerosol forming materials, binders, ovoid elements, irregularly shaped elements, shredded pieces, flakes, elements including tobacco, elements including a visible aerosol-forming material, adsorbent objects, absorbent objects, capsules, microcapsules, a honeycomb monolith, a single porous structure, and combinations thereof.
Embodiment 26: The method of any preceding or subsequent embodiment, or combinations thereof, further comprising inserting a first separating element in the second tubular member between the first aerosol generation arrangement and the second aerosol generation arrangement, the first separating element being one of heat-conductive and air permeable.
Embodiment 27: The method of any preceding or subsequent embodiment, or combinations thereof, further comprising inserting a second separating element in the second tubular member between the second aerosol generation arrangement and the mouth-engaging end of the cartridge body portion, wherein the second separating element is one of heat-conductive and air permeable.
Embodiment 28: The method of any preceding or subsequent embodiment, or combinations thereof, wherein the second aerosol generation arrangement comprises a cartridge having an elongate tubular body and opposed end members, wherein each of the end members is one of heat-conductive and air permeable, wherein the elongate tubular body is further configured to receive at least one aerosol-generating element and to cooperate with the opposed end members to contain the at least one aerosol-generating element therein, and wherein inserting the second aerosol generation arrangement further comprises inserting the cartridge within the second tubular member of the cartridge body portion.
Embodiment 29: A method of forming an aerosol delivery system, said method comprising: engaging one end of a first elongate tubular member with a first end of a second tubular member, the first elongate tubular member being configured as a control body portion and having a power source disposed therein, and the second tubular member being configured as a cartridge body portion and having a first aerosol generation arrangement disposed therein, the first aerosol generation arrangement being configured to operably engage the power source upon engagement between the one end of the control body portion and the first end of the cartridge body portion, the second end of the cartridge body portion facing toward a mouth-engaging end of the aerosol delivery system; and engaging a second aerosol generation arrangement with the cartridge body portion such that the second aerosol generation arrangement is disposed between the first aerosol generation arrangement and the mouth-engaging end of the aerosol delivery system.
Embodiment 30: The method of any preceding or subsequent embodiment, wherein the second aerosol generation arrangement comprises a plurality of aerosol-generating elements in the form of beads or pellets retained in place by a first air permeable separating element disposed within the second tubular member between the first aerosol generation arrangement and the second aerosol generation arrangement and a second separating element between the second aerosol generation arrangement and the mouth-engaging end.
Embodiment 31: The method of any preceding or subsequent embodiment, wherein said step of engaging a second aerosol generation arrangement with the cartridge body portion comprises removably engaging the second aerosol generation arrangement with the cartridge body portion, the second aerosol generation arrangement comprising a first end configured to removably engage with the cartridge body portion and a second end adapted to provide the mouth-engaging end of the aerosol delivery system, and wherein the second aerosol generation arrangement comprises a plurality of aerosol-generating elements in the form of beads or pellets retained in place by a first air permeable separating element between the first aerosol generation arrangement and the second aerosol generation arrangement and a second separating element between the second aerosol generation arrangement and the mouth-engaging end.
Embodiment 32: The method of any preceding or subsequent embodiment, wherein the second aerosol generation arrangement comprises a plurality of aerosol-generating elements in the form of beads or pellets comprising at least one aerosol forming material.
Embodiment 33: The method of any preceding or subsequent embodiment, wherein the aerosol-generating elements further comprise one or more of particulate tobacco, a tobacco extract, and nicotine, wherein the nicotine in free base form, salt form, as a complex, or as a solvate.
Embodiment 34: The method of any preceding or subsequent embodiment, wherein the aerosol-generating elements further comprise one or more fillers, binders, flavorants, and combinations thereof.
Embodiment 35: The method of any preceding or subsequent embodiment, wherein the aerosol-generating elements are smoke-treated.

These and other features, aspects, and advantages of the present disclosure will be apparent from a reading of the following detailed description together with the accompanying drawings, which are briefly described below. The present disclosure includes any combination of two, three, four, or more of the above-noted aspects as well as combinations of any two, three, four, or more features or elements set forth in this disclosure, regardless of whether such features or elements are expressly combined in a specific embodiment description herein. This disclosure is intended to be read holistically such that any separable features or elements of the present disclosure, in any of its various aspects and embodiments, should be viewed as intended to be combinable unless the context clearly dictates otherwise.

### BRIEF DESCRIPTION OF THE DRAWINGS

Having thus described the disclosure in general terms, reference will now be made to the accompanying drawings, which are not necessarily drawn to scale, and wherein:
FIG. 1 schematically illustrates an aerosol delivery device comprising a cartridge body and a control body, the cartridge body being illustrated in an exploded configuration and the control body being illustrated in an assembled configuration according to an example aspect of the present disclosure;
FIG. 2 schematically illustrates the control body of FIG. 1 in an exploded configuration according to an example aspect of the present disclosure;
FIG. 3 schematically illustrates the cartridge body of FIG. 1 implementing an additional aerosol generation arrangement, including one or more aerosol-generating elements, according to one aspect of the present disclosure;
FIG. 4 schematically illustrates the additional aerosol generation arrangement of FIG. 3, configured as a cartridge including one or more aerosol-generating elements, according to another aspect of the present disclosure;
FIG. 5 schematically illustrates an exploded view of an alternate carbon-based cartridge body according to an example aspect of the present disclosure;
FIG. 6A schematically illustrates an assembled view of the carbon-based cartridge body of FIG. 5, according to an example aspect of the present disclosure;
FIG. 6B schematically illustrates an assembled view of the carbon-based cartridge body, implementing an additional aerosol generation arrangement, including one or more aerosol-generating elements, according to one aspect of the present disclosure;
FIG. 7 is a cross-sectional view of a second aerosol generation arrangement housed within the same outer body as a first aerosol generation arrangement according to an example aspect of the present disclosure; and
FIG. 8 is a cross-sectional view of a second aerosol generation arrangement removably attached to the outer body housing a first aerosol generation arrangement according to an example aspect of the present disclosure.

### DETAILED DESCRIPTION OF THE PREFERRED EMBODIMENTS

The present disclosure will now be described more fully hereinafter with reference to exemplary embodiments thereof. These exemplary embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements. As used in the specification, and in the appended claims, the singular forms "a", "an", "the", include plural variations unless the context clearly dictates otherwise.

As described hereinafter, aspects of the present disclosure relate to aerosol delivery systems. Aerosol delivery systems according to the present disclosure use electrical energy to heat a material (preferably without combusting the material to any significant degree) to form an inhalable substance; and components of such systems have the form of articles most preferably sufficiently compact for such systems to be considered hand-held devices. That is, use of components of preferred aerosol delivery systems does not result in the production of smoke in the sense that aerosol results principally from by-products of combustion or pyrolysis of tobacco, but rather, use of those preferred systems results in the production of vapors (including vapors within aerosols that can be considered to be visible/not visible aerosols that might be considered to be described as smoke-like), resulting from volatilization or vaporization of certain components incorporated therein. In preferred aspects, components of aerosol delivery systems may be characterized as electronic cigarettes, and those electronic cigarettes most preferably incorporate tobacco and/or components derived from tobacco, and hence deliver tobacco derived components in aerosol form.

Aerosol generating pieces of certain preferred aerosol delivery systems may provide many of the sensations (e.g., inhalation and exhalation rituals, types of tastes or flavors, organoleptic effects, physical feel, use rituals, visual cues such as those provided by visible aerosol, and the like) of smoking a cigarette, cigar, or pipe that are provided by lighting and burning tobacco (and hence inhaling tobacco smoke), without any substantial degree of combustion of any component thereof. For example, the user of an aerosol generating piece of the present disclosure can hold and use that piece much like a smoker employs a traditional type of smoking article, draw on one end of that piece for inhalation of aerosol produced by that piece, take or draw puffs at selected intervals of time, and the like.

Aerosol delivery systems of the present disclosure also can be characterized as being suitable vapor-producing articles, aerosol-producing articles, or medicament delivery articles. Thus, such articles, systems, or devices can be adapted so as to provide one or more substances (e.g., flavors, pharmaceutical active ingredients, peptides, protein fragments, and/or protein coats) in an inhalable form or state. For example, inhalable substances can be substantially in the form of a vapor (i.e., a substance that is in the gas phase at a temperature lower than its critical point). Alternatively, inhalable substances can be in the form of an aerosol (i.e., a suspension of fine solid particles or liquid droplets in a gas). For purposes of simplicity, the term "aerosol" as used herein is meant to include vapors, gases, aerosols, and/or particulate matter of a form or type suitable for human inhalation, whether or not visible, and whether or not of a form that might be considered to be smoke-like.

Aerosol delivery systems of the present disclosure most preferably comprise some combination of a power source (i.e., an electrical power source), at least one control component (e.g., means for actuating, controlling, regulating and/or ceasing power supplied for heat generation, such as by controlling electrical current flow from an electrical power release unit to other components of the aerosol generating arrangement), a heater or heat generation component (e.g., an electrical resistance heating element and related components commonly referred to as providing an "atomizer"), and an aerosol precursor composition (e.g., a composition that commonly is a liquid capable of yielding an aerosol upon application of sufficient heat, such as ingredients commonly referred to as "smoke juice," "e-liquid" and "e-juice"), and a mouth end region, mouth-engaging end, or tip for allowing draw upon the aerosol delivery system for aerosol inhalation (e.g., a defined air flow path through the aerosol generation arrangement such that aerosol generated can be withdrawn therefrom upon draw).

More specific formats, configurations and arrangements of components within the aerosol delivery systems of the present disclosure will be evident in light of the further disclosure provided hereinafter. Additionally, the selection and arrangement of various aerosol delivery system components can be appreciated upon consideration of the commercially available electronic aerosol delivery devices, such as those representative products referenced in background art section of the present disclosure.

In some aspects, the use of aerosol delivery devices of the present disclosure may be subjected to many of the physical actions employed by an individual in using a traditional type of smoking article (e.g., a cigarette, cigar or pipe that is employed by lighting and inhaling tobacco). For example, the user of an aerosol delivery device of the present disclosure can hold that article much like a traditional type of smoking article, draw on one end of that article for inhalation of aerosol produced by that article, take puffs at selected intervals of time, or for selected durations of time, etc.

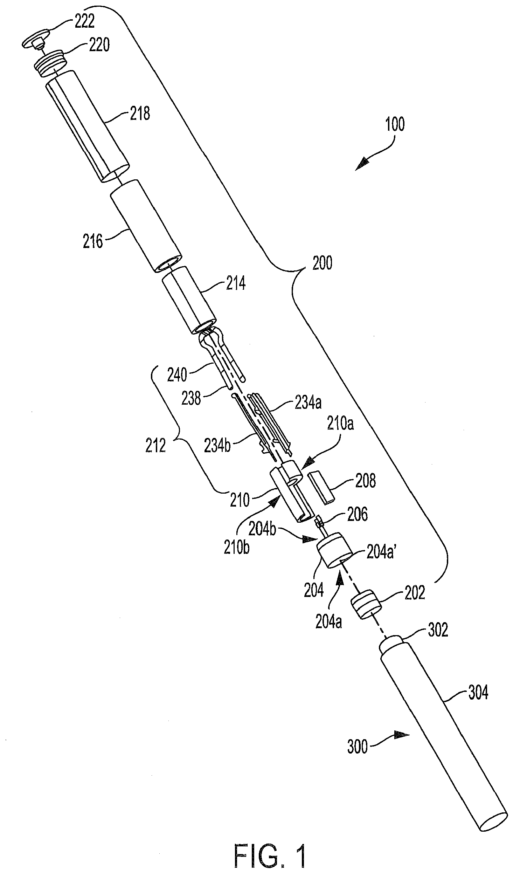

One such example of an aerosol delivery system 100 is illustrated in FIG. 1. In particular, FIG. 1 illustrates a partially exploded view of an aerosol delivery system 100 including a cartridge body 200 and a control body 300 (otherwise referred to herein as "cartridge body portion" and "control body portion," respectively). The cartridge body 200 and the control body 300 can be permanently or detachably aligned, or removably engaged, in a functioning relationship. Various mechanisms may be used to connect the cartridge body 200 to the control body 300 to result in a threaded engagement, a press-fit engagement, an interference fit, a magnetic engagement, or the like. The aerosol delivery system 100 may be substantially rod-like, substantially tubular shaped, or substantially cylindrically shaped in some embodiments, when the cartridge body 200 and the control body 300 are in an assembled configuration. As used herein, "tubular" is intended to refer to a hollow, elongated body, but is not limited to a specific cross-sectional shape or to a specific outer contour of the body. One skilled in the art will also appreciate that, in some instances and though not described in detail herein, the cartridge body 200 and the control body 300 forming the aerosol delivery system 100 may be configured in a single-piece, non-detachable form and may incorporate the components, aspects, and features associated with and disclosed in the present disclosure.

In some instances, one or both of the cartridge body 200 and the control body 300 may be referred to as being disposable (i.e., the single piece, non-detachable form previously disclosed) or as being reusable. For example, a reusable control body 300 may have a replaceable battery or a rechargeable battery and thus may be combined with any type of recharging technology, including connection to a typical alternating current electrical outlet, connection to a car charger (i.e., cigarette lighter receptacle), and connection to a computer, such as through a universal serial bus (USB) cable. In general, an aerosol delivery system of the type disclosed herein incorporates a battery or other electrical power source to provide current flow sufficient to provide various functionalities to the article, such as powering of a heater or heating element, powering of control systems, powering of indicators, and the like. The power source can take on various embodiments. Preferably, the power source is able to deliver sufficient power to rapidly heat the heating element to provide for aerosol formation and power the article through use for the desired duration of time. The power source preferably is sized to fit conveniently within the aerosol delivery device/system so that the aerosol delivery device/system can be easily handled; and additionally, a preferred power source is of a sufficiently light weight to not detract from a desirable smoking experience. Further, in some instances, the cartridge body 200 may comprise a single-use cartridge (i.e., disposable), as disclosed, for example, in U.S. Pat. App. Pub. No. 2014/0060555 to Chang et al..

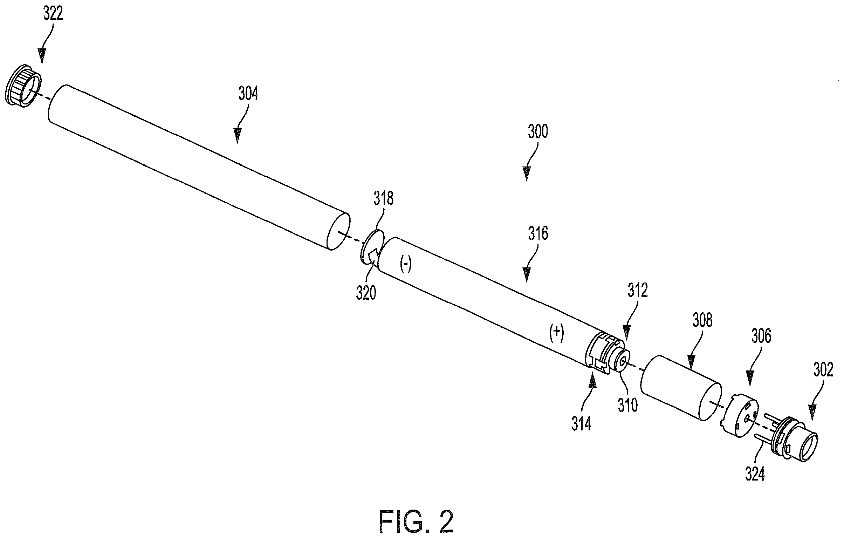

FIG. 2 illustrates an exploded view of the control body 300 of the aerosol delivery system 100 according to another example. As illustrated, the control body 300 may comprise a coupler 302, an outer body 304, a sealing member 306, an adhesive member 308 (e.g., KAPTON^{®} tape), a flow sensor 310 (e.g., a puff sensor or pressure switch), a control component 312, a spacer 314, an electrical power source 316 (e.g., a battery, which may be rechargeable), a circuit board with an indicator 318 (e.g., a light emitting diode (LED)), a connector circuit 320, and an end cap 322. Examples of electrical power sources are described in U.S. Pat. App. Pub. No. 2010/0028766 by Peckerar et al..

With respect to the flow sensor 310, representative current regulating components and other current controlling components including various microcontrollers, sensors, and switches for aerosol delivery devices/systems are described, for example, in U.S. Pat. Nos. 4,735,217 to Gerth et al.; 4,947,874 to Brooks et al.; 5,372,148 to McCafferty et al.; 6,040,560 to Fleischhauer et al.; 7,040,314 to Nguyen et al. and 8,205,622 to Pan; U.S. Pat. Pub. Nos. 2009/0230117 to Fernando et al.; 2014/0060554 to Collett et al..

In some instances, the indicator 318 may comprise one or more light emitting diodes. The indicator 318 can be in communication with the control component 312 through the connector circuit 320 and illuminate, for example, during a user drawing on a cartridge body 200 coupled to the coupler 302, as detected by the flow sensor 310. The end cap 322 may be adapted to make visible the illumination provided thereunder by the indicator 318. Accordingly, the indicator 318 may illuminate during use of the aerosol delivery system 100 to simulate the lit end of a smoking article. However, in other instances, the indicator 318 can be provided in varying numbers and can take on different shapes and can even be an opening in the outer body (such as for release of sound when such indicators are present). Additional representative types of components that yield visual cues or indicators, such as light emitting diode (LED) components, and the configurations and uses thereof, are described in U.S. Pat. Nos. 5,154,192 to Sprinkel et al.; 8,499,766 to Newton and 8,539,959 to Scatterday.

Still further features, controls or components that can be incorporated into aerosol delivery devices and systems of the present disclosure are described in U.S. Pat. Nos. 5,967,148 to Harris et al.; 5,934,289 to Watkins et al.; U.S. Pat. No. 5,954,979 to Counts et al.; 6,040,560 to Fleischhauer et al.; 7,726,320 to Robinson et al.; 8,365,742 to Hon; 8,402,976 and 8,689,804 to Fernando et al.; U.S. Pat. App. Pub. Nos. 2013/0192623 to Tucker et al.; 2013/0298905 to Leven et al.; and 2014/0000638 to Sebastian et al..

Returning to FIG. 1, the cartridge body 200 is illustrated in an exploded configuration. As illustrated, the cartridge body 200 may comprise a base shipping plug 202, a base 204, a control component terminal 206, an electronic control component 208, a flow tube 210, an atomizer 212, a reservoir substrate 214, an outer body 216, a label 218, a mouthpiece 220, and a mouthpiece shipping plug 222 according to an example embodiment of the present disclosure. The base 204 may be coupled to a first end of the outer body 216 and the mouthpiece 220 may be coupled to an opposing second end of the outer body 216 to enclose the remaining components of the cartridge body 200 therein. The base 204 may be configured to removably engage the coupler 302 of the control body 300. In some instances, the base 204 may comprise anti-rotation features that substantially prevent relative rotation between the cartridge body and the control body.

The base shipping plug 202 may be configured to engage and protect the base 204 prior to use of the cartridge body 200. Similarly, the mouthpiece shipping plug 222 may be configured to engage and protect the mouthpiece 220 prior to use of the cartridge body 200. The control component terminal 206, the electronic control component 208, the flow tube 210, the atomizer 212, and the reservoir substrate 214 (engaging the aerosol precursor composition or substance) may be retained within the outer body 216. The label 218 may at least partially surround the outer body 216 and include information such as a product identifier thereon.

Alignment of the components within either or both of the control body and the cartridge body of the aerosol delivery device/system can vary. In particular aspects, the aerosol precursor composition can be located near one end of the overall article (e.g., within a cartridge body, which in certain circumstances can be replaceable and disposable), which may be configured to be positioned in relatively closer proximity to the mouth of a user so as to maximize aerosol delivery to the user. Other configurations, however, are not excluded. Generally, the heating element can be positioned sufficiently near the aerosol precursor composition so that heat from the heating element can volatilize the aerosol precursor (and/or one or more flavorants, medicaments, or the like that may likewise be provided for delivery to a user) and form an aerosol for delivery to the user. When the heating element heats the aerosol precursor composition, an aerosol is formed, released, or generated in a physical form suitable for inhalation by a consumer. It should be noted that the foregoing terms are meant to be interchangeable such that reference to release, releasing, releases, or released includes form or generate, forming or generating, forms or generates, and formed or generated. Specifically, an inhalable substance is released in the form of a vapor or aerosol or mixture thereof. Additionally, the selection of various aerosol delivery device components can be appreciated upon consideration of the commercially available electronic aerosol delivery devices, such as those representative products listed above in the present disclosure.

The atomizer (i.e., an aerosol generation arrangement) 212 may comprise a first heating terminal 234a and a second heating terminal 234b, a liquid transport element 238 and a heating element 240. In this regard, the reservoir and/or reservoir substrate 214 may be configured to hold an aerosol precursor composition. The aerosol precursor composition, also referred to as a vapor precursor composition, may comprise a variety of components in different aspects. Such components may include, by way of example, any of a polyhydric alcohol (e.g., glycerin, propylene glycol, or a mixture thereof), nicotine, tobacco, tobacco extract, water, flavorants, and combinations thereof.

The aerosol precursor, or vapor precursor composition, can vary. Most preferably, the aerosol precursor composition is comprised of a combination or mixture of various ingredients or components. The selection of the particular aerosol precursor components, and the relative amounts of those components used, may be altered in order to control the overall chemical composition of the mainstream aerosol produced by the aerosol generation arrangement(s). Of particular interest are aerosol precursor compositions that can be characterized as being generally liquid in nature. For example, representative generally liquid aerosol precursor compositions may have the form of liquid solutions, viscous gels, mixtures of miscible components, or liquids incorporating suspended or dispersed components. Typical aerosol precursor compositions are capable of being vaporized upon exposure to heat under those conditions that are experienced during use of the aerosol generation arrangement(s) that are characteristic of the present disclosure; and hence are capable of yielding vapors and aerosols that are capable of being inhaled.

For aerosol delivery systems that are characterized as electronic cigarettes, the aerosol precursor composition most preferably incorporates tobacco or components derived from tobacco. In one regard, the tobacco may be provided as parts or pieces of tobacco, such as finely ground, milled or powdered tobacco lamina. In another regard, the tobacco may be provided in the form of an extract, such as a spray dried extract that incorporates many of the water soluble components of tobacco. Alternatively, tobacco extracts may have the form of relatively high nicotine content extracts, which extracts also incorporate minor amounts of other extracted components derived from tobacco. In another regard, components derived from tobacco may be provided in a relatively pure form, such as certain flavoring agents that are derived from tobacco. In one regard, a component that is derived from tobacco, and that may be employed in a highly purified or essentially pure form, is nicotine (e.g., pharmaceutical grade nicotine).

As noted above, highly purified tobacco-derived nicotine (e.g., pharmaceutical grade nicotine having a purity of greater than 98% or greater than 99%) or a derivative thereof can be used in the present invention. Representative nicotine-containing extracts can be provided using the techniques set forth in U.S. Pat. No. 5,159,942 to Brinkley et al.*,*. In certain embodiments, the products of the invention can include nicotine in any form from any source, whether tobacco-derived or synthetically-derived. Nicotinic compounds used in the products of the invention can include nicotine in free base form, salt form, as a
complex, or as a solvate. See, for example, the discussion of nicotine in free base form in U.S. Pat. Pub. No. 2004/0191322 to Hansson. At least a portion of the nicotinic compound can be employed in the form of a resin complex of nicotine where nicotine is bound in an ion exchange resin such as nicotine polacrilex. See, for example, U.S. Pat. No. 3,901,248 to Lichtneckert et al.. At least a portion
of the nicotine can be employed in the form of a salt. Salts of nicotine can be provided using the types of ingredients and techniques set forth in U.S. Pat. No. 2,033,909 to Cox et al. and Perfetti, Beitrage Tabalforschung Int., 12,43-54 (1983). Additionally, salts of nicotine have been available from sources such as Pfaltz and Bauer, Inc. and K&K Laboratories, Division of ICN Biochemicals, Inc. Exemplary pharmaceutically acceptable nicotine salts include nicotine salts of tartrate (e.g., nicotine tartrate and nicotine bitartrate), chloride (e.g., nicotine hydrochloride and nicotine dihydrochloride), sulfate, perchlorate, ascorbate, fumarate, citrate, malate, lactate, aspartate, salicylate, tosylate, succinate, pyruvate, and the like; nicotine salt hydrates (e.g., nicotine zinc chloride monohydrate), and the like. In certain embodiments, at least a portion of the nicotinic compound is in the form of a salt with an organic acid moiety, including, but not limited to, levulinic acid as discussed in U.S. Pat. Pub. No. 2011/0268809 to Brinkley et al..

The aerosol precursor composition may also incorporate so-called "aerosol forming materials." Such materials may, in some instances, have the ability to yield visible (or not visible) aerosols when vaporized upon exposure to heat under those conditions experienced during normal use of aerosol generation arrangement(s) that are characteristic of the present disclosure. Such aerosol forming materials include various polyols or polyhydric alcohols (e.g., glycerin, propylene glycol, and mixtures thereof). Aspects of the present disclosure also incorporate aerosol precursor components that can be characterized as water, saline, moisture or aqueous liquid. During conditions of normal use of certain aerosol generation arrangement(s), the water incorporated within those aerosol generation arrangement(s) can vaporize to yield a component of the generated aerosol. As such, for purposes of the current disclosure, water that is present within the aerosol precursor composition may be considered to be an aerosol forming material.

It is possible to employ a wide variety of optional flavoring agents or materials that alter the sensory character or nature of the drawn mainstream aerosol generated by the aerosol delivery system of the present disclosure. For example, such optional flavoring agents may be used within the aerosol precursor composition or substance to alter the flavor, aroma and organoleptic properties of the aerosol. Certain flavoring agents may be provided from sources other than tobacco. Exemplary flavoring agents may be natural or artificial in nature, and may be employed as concentrates or flavor packages.

Exemplary flavoring agents include vanillin, ethyl vanillin, cream, tea, coffee, fruit (e.g., apple, cherry, strawberry, peach and citrus flavors, including lime and lemon), maple, menthol, mint, peppermint, spearmint, wintergreen, nutmeg, clove, lavender, cardamom, ginger, honey, anise, sage, cinnamon, sandalwood, jasmine, cascarilla, cocoa, licorice, and flavorings and flavor packages of the type and character traditionally used for the flavoring of cigarette, cigar and pipe tobaccos. Syrups, such as high fructose corn syrup, also can be employed. Certain flavoring agents may be incorporated within aerosol forming materials prior to formulation of a final aerosol precursor mixture (e.g., certain water soluble flavoring agents can be incorporated within water, menthol can be incorporated within propylene glycol, and certain complex flavor packages can be incorporated within propylene glycol). However, in some aspects of the present disclosure, the aerosol precursor composition is free of any flavorants, flavor characteristics or additives.

Aerosol precursor compositions also may include ingredients that exhibit acidic or basic characteristics (e.g., organic acids, ammonium salts or organic amines). For example, certain organic acids (e.g., levulinic acid, succinic acid, lactic acid, and pyruvic acid) may be included in an aerosol precursor formulation incorporating nicotine, preferably in amounts up to being equimolar (based on total organic acid content) with the nicotine. For example, the aerosol precursor may include about 0.1 to about 0.5 moles of levulinic acid per one mole of nicotine, about 0.1 to about 0.5 moles of succinic acid per one mole of nicotine, about 0.1 to about 0.5 moles of lactic acid per one mole of nicotine, about 0.1 to about 0.5 moles of pyruvic acid per one mole of nicotine, or various permutations and combinations thereof, up to a concentration wherein the total amount of organic acid present is equimolar to the total amount of nicotine present in the aerosol precursor composition. However, in some aspects of the present disclosure, the aerosol precursor composition is free of any acidic (or basic) characteristics or additives.

As one non-limiting example, a representative aerosol precursor composition or substance can include glycerin, propylene glycol, water, saline, and nicotine, and combinations or mixtures of any or all of those components. For example, in one instance, a representative aerosol precursor composition may include (on a weight basis) about 70% to about 100% glycerin, and often about 80% to about 90% glycerin; about 5% to about 25% water, often about 10% to about 20% water; and about 0.1% to about 5% nicotine, often about 2% to about 3% nicotine. In one particular non-limiting example, a representative aerosol precursor composition may include about 84% glycerin, about 14% water, and about 2% nicotine. The representative aerosol precursor composition may also include propylene glycol, optional flavoring agents or other additives in varying amounts on a weight basis. In some instances, the aerosol precursor composition may comprise up to about 100% by weight of any of glycerin, water, and saline, as necessary or desired.

Representative types of aerosol precursor components and formulations also are set forth and characterized in U.S. Pat. No. 7,217,320 to Robinson et al. and U.S. Pat. Pub. Nos. 2013/0008457 to Zheng et al.; 2013/0213417 to Chong et al. and 2014/0060554 to Collett et al.. Other aerosol precursors that may be employed include the aerosol precursors that have been incorporated in the VUSE^{®} product by R.
J. Reynolds Vapor Company, the BLU^{™} product by Lorillard Technologies, the MISTIC MENTHOL product by Mistie Ecigs, and the VYPE product by CN Creative Ltd. Also desirable are the so-called "smoke juices" for electronic cigarettes that have been available from Johnson Creek Enterprises LLC.

The amount of aerosol precursor that is incorporated within the aerosol delivery system is such that the aerosol generation arrangement(s) provide acceptable sensory and desirable performance characteristics. For example, it is highly preferred that sufficient amounts of aerosol forming material (e.g., glycerin and/or propylene glycol), be employed in order to provide for the generation of a mainstream aerosol (visible or not visible) that in many regards resembles the appearance of tobacco smoke. The amount of the aerosol precursor composition within the aerosol generation arrangement(s) may be dependent upon factors such as the number of puffs desired per aerosol generation arrangement. Typically, the amount of the aerosol precursor composition incorporated within the aerosol delivery system, and particularly within the aerosol generation arrangement(s), is less than about 2 g, generally less than about 1.5 g, often less than about 1 g and frequently less than about 0.5 g.

The reservoir substrate 214 may comprise a plurality of layers of nonwoven fibers formed into the shape of a tube encircling the interior of the outer body 216 of the cartridge body 200. Thus, liquid components, for example, can be sorptively retained by the reservoir substrate 214. The reservoir substrate 214 is in fluid connection with the liquid transport element 238. The liquid transport element 238 may be configured to transport liquid (i.e., the aerosol precursor composition) from the reservoir substrate 214 to the heating element 240 via capillary action. Representative types of substrates, reservoirs or other components for supporting the aerosol precursor composition are described in U.S. Pat. No. 8,528,569 to Newton.

As illustrated, the liquid transport element 238 may be in direct contact with the heating element 240. As further illustrated in FIG. 1, the heating element 240 may comprise a wire defining a plurality of coils wound about the liquid transport element 238. In some instances, the heating element 240 may be formed by winding the wire about the liquid transport element 238. Further, in some instances, the wire may define variable coil spacing. Various materials configured to produce heat when an electrical current is applied thereto may be employed to form the heating element 240. Example materials from which the wire coil may be formed include Kanthal (FeCrAl), Nichrome, molybdenum disilicide (MoSi₂), molybdenum silicide (MoSi), molybdenum disilicide doped with aluminum (Mo(Si,Al)₂₎, graphite and graphite-based materials; and ceramic (e.g., a positive or negative temperature coefficient ceramic).

However, various other methods may be employed to form the heating element 240, and various other aspects of heating elements may be employed in the atomizer 212. For example, a stamped heating element may be employed in the atomizer. Further to the above, additional representative heating elements and materials for use therein are described in U.S. Pat. No. 5,060,671 to Counts et al.; U.S. Pat. No. 5,093,894 to Deevi et al.; U.S. Pat. No.5,224,498 to Deevi et al.; U.S. Pat. No. 5,228,460 to Sprinkel Jr., et al.; U.S. Pat. No. 5,322,075 to Deevi et al.; U.S. Pat. No. 5,353,813 to Deevi et al.; U.S. Pat. No. 5,468,936 to Deevi et al.; U.S. Pat. No. 5,498,850 to Das; U.S. Pat. No. 5,659,656 to Das; U.S. Pat. No. 5,498,855 to Deevi et al.; U.S. Pat. No. 5,530,225 to Hajaligol; U.S. Pat. No. 5,665,262 to Hajaligol; U.S. Pat. No. 5,573,692 to Das et al.; and U.S. Pat. No. 5,591,368 to Fleischhauer et al., the disclosures of which are incorporated herein by reference in their entireties. Further, chemical heating may be employed in other aspects. A variety of heater components may also be used in particular aspects of the present aerosol delivery device/system. In various instances, one or more microheaters or similar solid state heating elements may be used. Exemplary microheaters that may be utilized are further described herein. Further microheaters and atomizers incorporating microheaters suitable for use in the presently disclosed devices/systems are described in U.S. Pat. App. Pub. No. 2014/0060554 to Collett et al..

The first heating terminal 234a and the second heating terminal 234b (e.g., positive and negative terminals) at the opposing ends of the heating element 240 are configured to form an electrical connection (which may be a removable or detachable connection) with the control body 300 when the cartridge body 200 is connected thereto. Further, when the control body 300 is coupled to the cartridge body 200, the electronic control component 208 may form an electrical connection with the control body 300 through the control component terminal 206. The control body 300 may thus employ the electronic control component 208 to determine whether the cartridge 200 is genuine and/or perform other functions. Further, various examples of electronic control components and functions performed thereby are described in U.S. Pat. App. Pub. No. 2014/0096781 to Sears et al..

During use, a user may draw on the mouthpiece or mouth-engaging end 220 of the cartridge body 200 of the aerosol delivery system 100. This may pull airthrough an opening in the control body 300 and/or in the cartridge body 200. For example, in one instance, an opening may be defined between the coupler 302 and the outer body 304 of the control body 300. However, the flow of air may be received through other parts of the aerosol delivery device/system 100 in other aspects. As noted above, in some aspects the cartridge body 200 may
include the flow tube 210. The flow tube 210 may be configured to direct the flow of air received from the control body 300 to the heating element 240 of the atomizer 212.

A sensor in the aerosol delivery device/system 100 (e.g., a puff or flow sensor in the control body 300) may sense the puff. More generally, a sensor or detector may be implemented to control of supply of electric power to the heating element 240 when aerosol generation is desired (e.g., upon draw during use). As such, for example, there is provided a manner or method for turning off the power supply to the heating element 240 when the aerosol generation is not desired during use, and for turning on the power supply to actuate or trigger the generation of heat by the heating element 240 during draw. Additional representative types of sensing or detection mechanisms, structure and configuration thereof, components thereof, and general methods of operation thereof, are described in U.S. Pat. Nos. 5,261,424 to Sprinkel, Jr.; 5,372,148 to McCafferty et al.; and PCT WO 2010/003480 by Flick. When the puff is sensed, the control body 300 may direct current to the heating element 240 through a circuit including the first heating terminal 234a and the second heating terminal 234b. Accordingly, the heating element
240 may vaporize the aerosol precursor composition directed to an aerosolization zone from the reservoir substrate 214 by the liquid transport element 238. Thus, the mouthpiece 220 may allow passage of air and entrained vapor (i.e., the components of the aerosol precursor composition in an inhalable form, for example, as an aerosol) from the cartridge body 200 to a consumer drawing thereon. Various other details with respect to the components that may be included in the cartridge body 200, are provided.

Various components of an aerosol delivery device/system can be chosen from components described in the art and commercially available. Reference is made for example to the reservoir and heater system for controllable delivery of multiple aerosolizable materials in an electronic smoking article disclosed in U.S. Pat. App. Pub. No. 2014/0000638 to Sebastian et al.. Note further that portions of the cartridge body 200 illustrated in FIG. 1 are optional. In this regard, by way of example, the cartridge body 200 may
not necessarily include the flow tube 210, the control component terminal 206, and/or the electronic control component 208, in some instances.

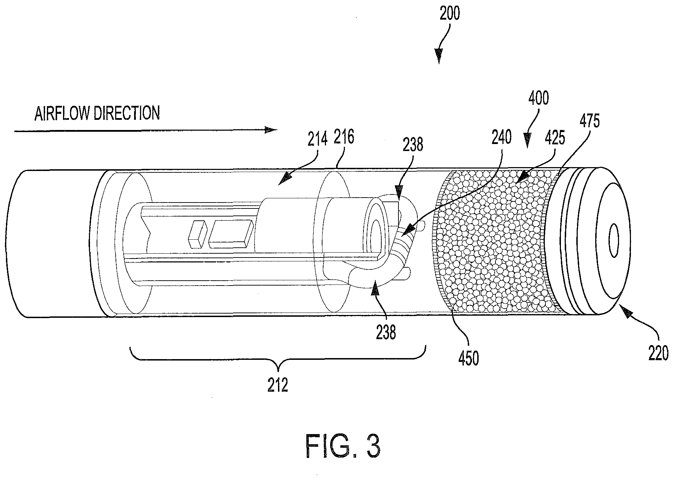

One particular aspect of the present disclosure is illustrated, for example, in FIG. 3. In such instances, the cartridge body 200 may further incorporate a second aerosol generation arrangement 400 (the atomizer 212 being considered "a first aerosol generation arrangement") disposed in the outer body 216, longitudinally between the atomizer 212 and the mouthpiece or mouth-engaging end 220 of the cartridge body 200. In some aspects, the second aerosol generation arrangement 400 is generally porous or otherwise configured to allow the passage of air therethrough. In some particular instances, the second aerosol generation arrangement 400 may include one or more aerosol-generating elements 425 that may be comprised of at least one or a plurality of pellets or beads or other appropriate elements or combinations thereof. In some instances, the at least one or a plurality of pellets or beads or other appropriate elements or combinations thereof forming the aerosol-generating element(s) 425 may be coaxially circumscribed by a generally tubular-shaped heat conductive member (not shown), if necessary, and/or circumscribed or otherwise jacketed by insulation (e.g., a non-woven mat or layer of glass filaments or fibers), or other suitable material (not shown).

The overall configuration of the second aerosol generation arrangement 400 within the cartridge body 200 of the aerosol delivery device/system 100 can be considered to be generally cylindrical in nature. Representative preferred beads or other objects may be produced from a formulation that incorporates tobacco (e.g., particulate tobacco), components of tobacco and/or materials that are otherwise derived from tobacco (e.g., tobacco extracts such as aqueous tobacco extracts or nicotine derived from tobacco including pharmaceutical grade nicotine). The beads most preferably incorporate flavors and a visible or non-visible aerosol forming material (e.g., glycerin or other material that generates a visible vapor that resembles smoke). That is, components of the beads are preferably configured to act as substrate components for volatile flavors, vapor forming materials, moisture or other liquid(s), and/or aerosol forming materials that are carried thereby. In some aspects, the aerosol-generating element(s) 425 may include or otherwise comprise or be configured as, for example, marumarized tobacco beads of varying shapes and sizes, a monolith of bonded (e.g., sintered) beads; a porous monolith; a single porous structure; a honeycomb monolith; a single piece of a porous material; beads of extruded tobacco; beads of porous material containing tobacco extract (e.g., calcium carbonate, ceramic, or the like); reconstituted tobacco shreds; expanded tobacco shreds; extruded rods of various materials (including hollow cylinders and slotted rods) containing tobacco flavors; shavings, granules, capsules, and/or microcapsules of various materials containing tobacco flavors or other substances, whether in a liquid or other form; and treatments or combinations thereof.

In general, as used herein, the terms "pellets" and "beads" are meant to include beads, pellets, or other discrete small units or pieces of that may include (in addition to those otherwise disclosed herein), for example, carbon pieces, extruded carbon pieces cut into pellets, ceramic beads, marumarized tobacco pieces, and the like, or combinations thereof. For example, granules, pellets or beads can be generally cylindrical or spherical extruded or compressed granules, pellets or beads comprised of a moistened mixture or slurry of milled tobacco lamina, fillers (e.g., granular calcium carbonate), flavors, visible aerosol forming materials and binders (e.g., carboxy methylcellulose) that are formed, cut or spun to the desired size and shape, and then dried to retain the desired configuration. However, such "pellets" or "beads" may comprise any suitable elements, or combination of elements, meeting the preferred aspects as disclosed herein. For example, some or all of the beads or pellets can comprise spherical capsules that are heat sensitive, so that when included in the aerosol-generating element and exposed to heat, the rupture or decomposition thereof causes the release of glycerin, propylene glycol, water, saline, tobacco flavor and/or nicotine or other substances or additives. Also, the beads can comprise ceramic or absorbent clay or silica or absorbent carbon to hold and release an aerosol former. Further, in some aspects, the beads/pellets may comprise a heat conductive material such as, for example, heat conductive graphite, heat conductive ceramic, a metal, tobacco cast on foil, a metal or other suitable material impregnated with appropriate aerosol-generating substances such as glycerin and flavor(s), or a suitable cast sheet material appropriately formed into the desired beads/pellets.

In one particular example, the beads/pellets (particles) may be comprised, by weight, of between about 15% and about 60% of finely milled tobacco particles (e.g., a blend of Oriental, burley and flue-cured tobaccos, essentially all Oriental tobacco, essentially all burley tobacco, or essentially all flue-cured tobacco), between about 15% and about 60% of finely milled particles of calcium carbonate (or finely milled clay or ceramic particles), between about 10% and about 50% of glycerol (and optionally a minor amount of flavors), between about 0.25% and about 15% of a binder (preferably carboxymethylcellulose, guar gum, potassium, or ammonium alginate), and between about 15% and about 50% of water. In another example, the beads/pellets (particles) may be comprised of about 30% of finely milled tobacco particles (e.g., a blend of Oriental, burley and flue-cured tobaccos, essentially all Oriental tobacco, essentially all burley tobacco, or essentially all flue-cured tobacco), about 30% of finely milled particles of calcium carbonate (or finely milled clay or ceramic particles), about 15% of glycerol (and optionally a minor amount of flavors), about 1% of a binder (preferably carboxymethylcellulose, guar gum, potassium, or ammonium alginate), and about 25% of water. In such examples, the particles may be compressed to hold the glycerol and, upon compression, may form a porous matrix that facilitates migration of the aerosol generating components to promote efficient aerosol formation. The manner by which the aerosol forming material is contacted with the substrate material can vary. The aerosol forming material can be applied to a formed material, can be incorporated into processed materials during manufacture of those materials, or can be endogenous to that material. Aerosol-forming material, such as glycerin, can be dissolved or dispersed in an aqueous liquid, or other suitable solvent or liquid carrier, and sprayed onto that substrate material. See, for example, U.S. Patent Appl. Pub. No. 2005/0066986 to Nestor et al. and 2012/0067360 to Conner et al.. The calcium carbonate or other inorganic filler assists in creating porosity within the particles, and may also function to absorb heat which may, in some instances limit or otherwise prevent scorching of the aerosol generating components, as well as assisting in and
promoting aerosol formation. See also, for example, those types of materials set forth in U.S. Pat. No. 5,105,831 to Banerjee, et al., and U.S. Pat. App. Pub. Nos. 2004/0173229 to Crooks et al.; 2011/0271971 to Conner et al.; and 2012/0042885 to Stone et al..

In one embodiment, the aerosol-generating elements 425, such as those in the form of beads or pellets, can be smoke-treated to impart smoky flavor or aroma. For example, the beads or pellets can be prepared and then subjected to smoke from a combustible source, such as a wood source (e.g., wood selected from hickory, maple, oak, apply, cherry, or mesquite). The beads or pellets can be treated with the smoke for a time sufficient to impart the desired smoky flavor or aroma, with an exemplary time range being about 5 to about 45 minutes. The manner in which the beads or pellets are contacted with smoke can vary, with one example involving heating wood chips in a container until smoke is produced (e.g., heating wood chips to a temperature of about 350-400°F) and placing the beads or pellets to be treated within a closed environment with the smoke produced by the wood chips.

The composition of the aerosol precursor composition of the first aerosol generation arrangement and the composition of the aerosol-generating elements of the second aerosol generation arrangement are advantageously selected so as to complement one another to produce a desirable sensory experience. In certain embodiments, for example, the nicotine content of the aerosol precursor composition and the aerosol-generating elements are selected such that either or both of the aerosol precursor composition and the aerosol-generating elements may contain nicotine or a nicotinic compound or may be viewed as substantially or completely free of nicotine or a nicotinic compound. In other words, all nicotine content can be within the aerosol-generating elements or all nicotine content can be in the aerosol precursor composition or both compositions can include nicotine in some form.

In some aspects, where the aerosol-generating elements 425 comprise, for example, beads or pellets cast or extruded from materials of the various types set forth above (i.e., a graphite bead including tobacco extract and glycerin), while "damp" or otherwise before drying, may be rolled, for example, between adjacent roller elements, to flatten the shape of the respective beads/pellets. In some instances, the materials of the various types set forth above may be extruded in the form of filamentary strands, wherein the strands may be gathered to form a cylindrical rod or other suitably shaped material (i.e., relative in size to the beads/pellets used to otherwise form the aerosol generation segment) for application in the second aerosol generation arrangement 400. Upon drying, the flattened beads/pellets may then be shredded or otherwise processed to form, for example, strands, flakes, or other filler configuration that is flat or includes a planar segment that inhibits or prevents roll. Any random configurations resulting from the shredding process may be sufficient. In such instances, the flattened and shredded beads/pellets may then be included in the aerosol-generating element(s) 425, and the irregular or random configurations thereof may promote, for instance, a plurality of interstitial air spaces throughout the aerosol-generating element(s) 425, wherein the interstitial air spaces may, in tum, promote heat transfer with the individual objects within the aerosol-generating element(s) 425. That is, heating of the air in the interstitial spaces within the second aerosol generation arrangement 400 may expose more of the aerosol-generating element(s) 425 to the heat from the heating element 240, and thus result in enhanced or otherwise improved heating of the aerosol-generating element(s) 425. In other instances, the heat and the first aerosol (i.e., the combination thereof) produced by the heating element 240 / atomizer 212 are directed through the porous matrix formed by the aerosol-generating element(s) 425, wherein the heated vapors passing through and heating the porous aerosol-generating element(s) 425 promotes, for example, elution (i.e., liquid, fluid, or particulate extraction; steam distillation; etc.) of an enhancement substance (i.e., a flavorant or other additive) from the aerosol-generating element(s) to the first aerosol, or otherwise promotes the enhancement substance being entrained in, imparted to, reacted with, or otherwise interacted with the first aerosol. The interaction between the enhancement substance and the first aerosol may, for example, change or alter the first aerosol, mix the enhancement substance with the first aerosol to form an enhanced aerosol or aerosol mixture, or facilitate a reaction that produces a different aerosol. In such instances, increased interstitial spaces within the aerosol-generating element(s) 425 may promote this interaction process through the second aerosol generation arrangement 400.

In some aspects, the beads / pellets may originate from a tobacco material cast on a foil/paper laminate. More particularly, the tobacco material may comprise, for example, a slurry including reconstituted tobacco, glycerin, and a binder material. Such a tobacco material is disclosed, for example, in U.S. Patent No. 5,101,839 to Jakob et al. and U.S. Patent Application No. 2010/0186757 to Crooks et al.. In addition, the slurry can incorporate granular inorganic material (i.e., calcium carbonate). The slurry is cast unto a paper element of a foil-paper laminate, such as disclosed, for example, in U.S. Patent No.8,678,013 to Crooks et al. and U.S. Patent No. 7,647,932 to Cantrell et al., and the assembled cast sheet product is then dried, for instance by the application of heat (i.e., by heated air, microwave drying, etc.). The paper element may have, for instance, a particular porosity or texture to promote an intimate contact and interaction with the slurry, for instance, over direct contact between the slurry and the foil. However, the exemplary aspect presented herein does not preclude casting the tobacco material (i.e., slurry) directly on a metal foil or other suitable thin film heat conductor. Once such a laminate is cast, the dried cast sheet (i.e., the foil/paper/tobacco material) may be shredded, diced, or otherwise separated into a plurality of cast sheet portion elements, wherein each such element preferably includes a portion of the tobacco material (i.e., the substrate) intimately interacted with a portion of the paper element which, in turn, is in intimate contact with a portion of the foil element of the foil-paper laminate. A plurality of the cast sheet portion elements may then be included in the aerosol-generating element(s) 425 forming the second aerosol generation arrangement 400.

One skilled in the art will appreciate that, in some circumstances, the cast sheet portion elements included in the aerosol-generating element(s) 425 may cooperate to promote improved heat transfer to the tobacco material forming a portion of those cast sheet portion elements or otherwise to abutting elements. More particularly, in some instances, heat transfer from the heating element 240 to the tobacco material included in the aerosol-generating element(s) 425 may be limited past any direct interface therebetween, with the heat-conducting strip forming an additional mechanism for conducting heat from the heating element 240 for heating the outer elements included in the aerosol-generating element(s) 425 and any aerosol-generating element(s) in contact therewith. In aspects including the cast sheet portion elements included in the aerosol-generating element(s) 425, the heat-conductive portions of the foil element associated with the cast sheet portion elements may form, for example, a plurality of additional heat conductive pathways. That is, the cast sheet portion elements used as all or part of the aerosol-generating element(s) 425 may provide additional heat-conductive elements interspersed throughout the aerosol-generating element(s) 425 within the second aerosol generation arrangement 400 to thereby enhance or otherwise improve heat transfer to and between the aerosol-generating elements. In achieving such an aspect, it may be further advantageous to shred or process a substrate material implemented in, for example, the cast tobacco sheet substrate material forming the substrate incorporated within the types of cigarettes commercially marketed under the trade name "Eclipse" by R. J. Reynolds Tobacco Company, as disclosed, e.g., by U.S. Patent No. 5,469.871 to Barnes et al.

The pellets or other elements may have smooth, regular outer shapes (e.g., spheres, cylinders, ovoids, or the like) and/or they may have irregular outer shapes (e.g., shredded pieces, flakes, or the like). The aerosol-generating element(s) 425, discretely or cumulatively, may have a generally cylindrical form within the second aerosol generation arrangement 400, and may in some instances include a collection of about 800 to about 1200 generally spherical beads, each having a mean or nominal diameter of about 0.05 mm to about 4 mm (e.g., about 1 mm³ in volume, in one example), with the beads/pellets cumulatively weighing about 450 mg to about 750 mg (e.g., 600 mg ± 25%, in one example).

In one method of preparation, substantially spherical beads or pellets of aerosol-generating elements can be formed by first mixing together the desired composition followed by extrusion of the composition to form an extrudate. The extrudate is then processed in a spheronizer (e.g., such as spheronizers available from Caleva Process Solutions Ltd. or LCI Corporation) to produce variously-sized spheroids that can be processed through a series of screens to provide the desired size range, such as the sizes noted above.

The aerosol-generating elements can be selected so as to have relatively uniform mean diameter or a range of sizes of aerosol-generating elements can be included in the second aerosol generation arrangement 400. Where different size ranges are used in the same device, the differently sized elements can be arranged in a gradient or layers within the second aerosol generation arrangement 400 or the differently sized elements can be randomly mixed within the aerosol generation arrangement 400. Although not bound by any particular theory of operation, using aerosol-generating elements of different sizes in the same aerosol generation arrangement 400 can provide advantageous pressure drop changes in the device and/or provide advantageous sensory characteristics based on the different rates of evaporation provided by the differently sized elements.

Preferably, sufficient beads are loaded into the second aerosol generation arrangement 400 to provide at least about 95 percent of maximum fill, with beads and/or other suitable elements. It is advantageous to avoid large open pockets within the aerosol generation arrangement 400 that could allow air traveling through the aerosol generation arrangement to substantially bypass interaction with the aerosol-generating elements 425.

In some instances, a plurality of forms of the aerosol-generating element(s) 425 may be selected (e.g., aerosol-generating element(s) having different compositions) and each selected form of the aerosol-generating elements then subsequently included in the second aerosol generation arrangement 400. In other instances, the selected forms of the aerosol-generating elements may be combined, prior to inclusion in the second aerosol generation arrangement 400, to produce an aerosol-generating element mixture, and the mixture then subsequently included in the second aerosol generation arrangement 400.

The atomizer or first aerosol generation arrangement 212 and the second aerosol generation arrangement 400 may be physically separate from one another and/or comprise discrete units or segments within the cartridge body 200. In some instances, as shown, those segments may be positioned/disposed so that the downstream end (toward the mouthpiece or mouth-engaging end 220 of the cartridge body 200) of the atomizer or first aerosol generation arrangement 212 is adjacent to the upstream end of the second aerosol generation segment 400 (i.e., the back face of the aerosol-generating element(s) 425). That is, the atomizer or first aerosol generation arrangement 212 and the second aerosol generation segment 400 may be axially aligned in a serial end-to-end relationship, in some instances adjacent to or abutting one another. For example, in some instances, though physically discrete and positioned downstream from the atomizer or first aerosol generation arrangement 212, it may be desirable for the aerosol-generating element(s) 425 of the second aerosol generation arrangement 400 to physically contact the heating element 240 at the downstream end of the atomizer or first aerosol generation arrangement 212. Alternatively, those segments 212, 400 can be slightly spaced apart from one another such that the respective ends or components thereof 240, 425 are not necessarily in physical contact with the other (i.e., to prevent scorching). One skilled in the art will appreciate that, in some aspects, the second aerosol generation arrangement 400 may comprise more than one section or portion of aerosol-generating element(s) 425.

In some instances, an additional segment, spacer element, or separating element (otherwise referred to herein as "a first separating element"), acting as a spacer or screen (see, e.g., element 450 in FIG. 3) may be positioned generally perpendicular to the longitudinal axis of the cartridge body 200, wherein the first separating element 450 may provide for physical separation of those two segments 212, 400 while, in some instances, maintaining a heat conductive relationship therebetween. The first separating element 450 may, in some instances, not be conductive to heat and, in other instances, the first separating element 450 may not be electrically conductive. That is, the first separating element 450 may, but not necessarily, be heat-conductive and/or arranged to conduct heat from the heating element 240 of the atomizer / first aerosol generation arrangement 212 to the second aerosol generation arrangement 400, wherein the aerosol-generating element(s) 425 may be responsive to the heat and/or accompanying first aerosol to form a second aerosol. Further, in some instances, the first separating element 450 may be air permeable or otherwise configured to permit airflow therethrough, such that a first aerosol generated by the atomizer / first aerosol generation arrangement 400 can pass therethrough in the downstream direction. The first separating element 450 may thus also be configured and/or arranged so as to maintain the aerosol-generating element(s) 425 within the second aerosol generation arrangement 400 and separate from the atomizer/first aerosol generation arrangement 212. In still further instances, the first separating element 450 may be configured as a spacer (i.e., extending in a longitudinal direction along the cartridge body 200 so as to define a thickness) for separating the aerosol-generating element(s) 425 from the heating element 240 of the atomizer/first aerosol generation arrangement 212, for example, to minimize or prevent the aerosol-generating element(s) (i.e., beads) 425 from being scorched or burned by the heat from the heating element 240. In some instances, the first separating element 450 may also be configured as an insulator (i.e., not electrically conductive) to prevent short-circuiting of the heating element 240 in the event of contact therebetween.

Typically, the first separating element 450 is generally cylindrical or discoid in shape and of one piece construction, and is air permeable to allow the passage of drawn air through. The first separating element 450 may be heat conductive in nature, so that heat generated by the heating element 240 can be readily transported to the second aerosol generation arrangement 400. The length (thickness) of the first separating element 450 can vary, and typically extends from about less than 1 mm up to about 10 mm. In some instances, the relative longitudinal placement of the first separating element 450 within the outer body 216, spaces the interface of the first separating element 450 with the aerosol-generating element(s) 425 at between about 1 mm and up to about 20 mm (i.e., 7 mm in one example) away from the heating element 240. Typically, the first separating element 450 is comprised of a heat resistant material, such as a porous ceramic, a porous graphite material, a metal (i.e., stainless steel, brass, copper, etc.) mesh or screen, a high temperature-resistant plastic or the like. In some instances, the first separating element 450 may include, for example, longitudinally-extending air passageways formed during design /manufacture, drilled therethrough, or otherwise molded, extruded, printed (i.e., a 3D printed element using a 3D printer), or shaped into the spacer element during manufacture thereof. If desired, the first separating element 450 can incorporate catalytic materials, such as materials incorporating cerium or copper ions or oxides and/or salts of cerium and copper ions. See, for example, U.S. Patent Nos. 8,469,035 and 8,617,263 to Banerjee et al. and U. S. Pat. Appl. Pub. No. 2007/0215168 to Banerjee et al..

In instances where the aerosol-generating element(s) 425 may be circumscribed by an insulation layer, a layer of heat conductive material (e.g., a layer or strip comprised of metal foil) may be provided therebetween (not shown). That is, representative aerosol-generating element(s) 425 include a plurality of pellets and/or other appropriate elements that can be circumscribed along its length by a layer of strip of metal foil. A representative metal foil is, for example, aluminum foil having a thickness of about 0.01 mm to about 0.05 mm. Preferably, the metal foil extends along the entire length of the outer co-axial surface of the aerosol-generating element(s) 425; and it may be preferred that the metal foil extends over (i.e., at least partially overlaps) the first separating element 450. The heat conductive material can be provided by means other than the use of metal foil. For example, the layer of metal foil can be replaced by a metal mesh or screen. Alternatively, the metal foil can be replaced by a heat conductive fabric, such as a layer or sheet of graphite fibers or heat conductive ceramic fibers. Alternatively, the heat conductive material can be provided by application of a heat conductive ink, such as a coating of ink or paint that incorporates metal particles, graphite fibers, particles of heat conductive ceramic materials, or the like.

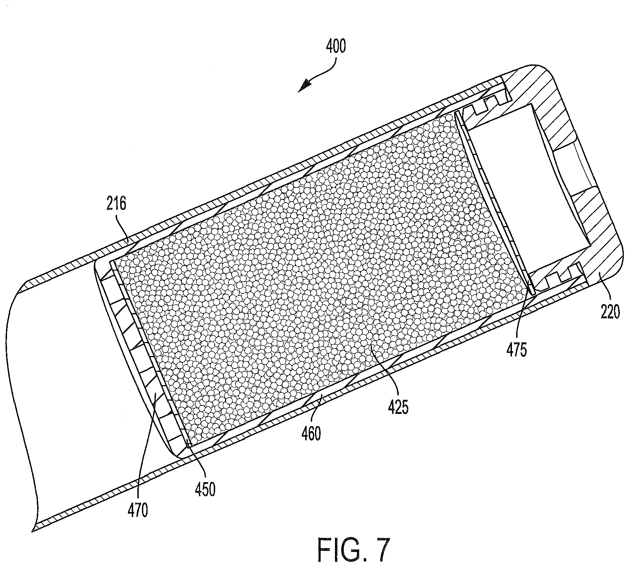

FIG. 7 provides an example of a further embodiment of a second aerosol generation arrangement 400 positioned within the outer body or tubular member 216 (downstream of the first aerosol generation arrangement 212, which is not shown). As shown, the aerosol-generating elements 425 are placed between separating elements, 450 and 475, which serve to retain the aerosol-generating elements 425 in place and to allow airflow therethrough. As noted previously, the separating elements, 450 and 475, can be porous elements (e.g., mesh screens or perforated metal plates) with pore sizes selected to as to retain the aerosol-generating elements 425 within the second aerosol generation arrangement 400.

As shown, the second aerosol generation arrangement 400 can further include a separate aerosol-generating element housing 460 in the form, for example, of a tubular housing with an open end facing the mouth-piece 220, which as shown, can engage the open end of the tubular housing and can be affixed thereto by a press fit or other known means. The housing 460 can include an end 470 opposite the mouthpiece 220, which as shown, can be perforated to allow airflow there through. The housing 460 is constructed of plastic. The separating elements, 450 and 475, can be press fit or otherwise engaged with the housing 460, and the separating element 475 closest to the mouthpiece 220 can be affixed to the mouthpiece if desired. In certain embodiments, the separating elements, 450 and 475, are incorporated into the housing 460 during the molding process that forms the housing. The design of FIG. 7 is particularly well-suited for embodiments of the invention wherein the second aerosol generation arrangement 400 is intended to be permanently affixed to the remainder of the cartridge body 200, rather than separately removable or disposable.

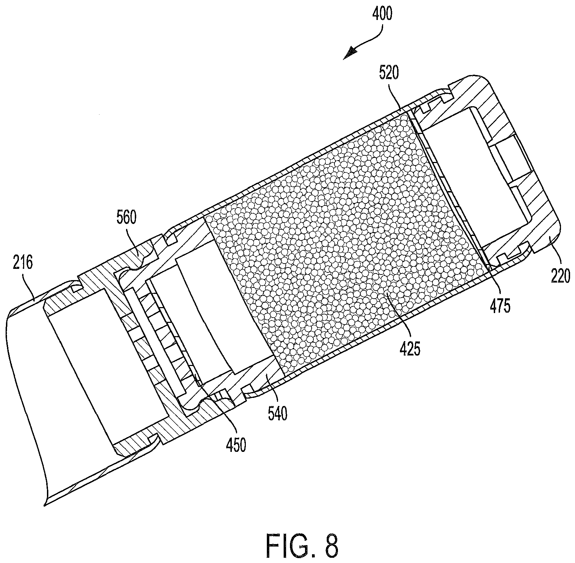

Alternatively, in embodiments where the second aerosol generation arrangement 400 is adapted for removal from the cartridge body 200 as a separate unit, the design of FIG. 8 is advantageous. As shown, in the embodiment of FIG. 8, the second aerosol generation arrangement 400 is formed as a separate unit with a separate housing body 520, which is attached (e.g., through crimping or other means) to a first connector 540. Together, the housing body 520 and first connector 540 form a cavity for the aerosol-generating elements 425. As with the embodiment of FIG. 7, the aerosol-generating elements 425 are placed between separating elements, 450 and 475, which serve to retain the aerosol-generating elements 425 in place and to allow airflow therethrough. Similar to the embodiment of FIG. 7, the separating elements, 450 and 475, can be press fit or otherwise engaged with the surrounding portions of the first connector 540 or housing body 520, respectively, and can be incorporated into these surrounding portions during a molding process. The downstream separating element 475 is also optionally affixed to the mouthpiece 220.

The first connector 540 of the second aerosol generation arrangement 400 is adapted for engagement with a second connector 560 that is affixed (e.g., through press fit or other means) to the outer body or tubular member 216 housing the first aerosol generation arrangement 212 (not shown). The second connector 560 has an end facing the first connector 540 that enables the user to removably affix the second aerosol generation arrangement 400 to the cartridge body 200, such as through a threaded engagement or other connection means. As shown, the second connector 560 is porous to allow airflow from the first aerosol generation arrangement 212 to enter the second aerosol generation arrangement 400. The second aerosol generation arrangement 400 of this embodiment is cooperatively engaged with the mouthpiece 220 in a manner similar to FIG. 7.

In some aspects, another spacer element, or another separating element (otherwise referred to herein as "a second separating element"), acting as a spacer or screen (see, e.g., element 475 in FIG. 3) may be positioned generally perpendicular to the longitudinal axis of the cartridge body 200, wherein the second separating element 475 may provide for physical separation of the second aerosol generation arrangement 400 from the mouthpiece or mouth-engaging end 220 of the cartridge body 200. That is, the second separating element 475 may, but not necessarily, be heat-conductive and/or arranged to conduct heat from the second aerosol generation arrangement 400 and through the mouthpiece or mouth-engaging end 220 of the cartridge body 200. However, the second separating element 475 may be air permeable or otherwise configured to permit airflow therethrough, such that a first aerosol generated by the atomizer / first aerosol generation arrangement 212 and/or a second aerosol generated by the second aerosol generation arrangement 400, can pass therethrough in the downstream direction and through the mouthpiece or mouth-engaging end 220 of the cartridge body 200. The second separating element 475 may thus also be configured and/or arranged so as to maintain the aerosol-generating element(s) 425 within the second aerosol generation arrangement 400, without loss of any of the aerosol-generating element(s) through the mouthpiece or mouth-engaging end 220 of the cartridge body 200.

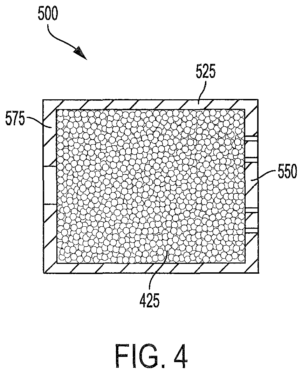

In some aspects, in the alternative to discrete first and second separating elements 450, 475 being implemented in addition to the aerosol-generating element(s) 425, the second aerosol generation arrangement 400 may comprise a cartridge 500 (see, e.g., FIG. 4) having an elongate tubular body 525 and opposed end members 550, 575, wherein each of the end members 550, 575 may be heat-conductive and/or air permeable in a similar manner to the first and second separating elements 450, 475. The elongate tubular body 525 is further configured to receive the aerosol-generating element(s) 425 and to cooperate with the opposed end members 550, 575 to contain the aerosol-generating element(s) 425 therein. The assembled cartridge 500 may thus be configured to be received as a unit (forming the second aerosol generation arrangement 400) by the outer body or tubular member 216 of the cartridge body 200.

In use, the mouthpiece or mouth-engaging end 220 of the cartridge body 200 of the aerosol delivery system 100 is inserted into the mouth of the user. The atomizer/first aerosol generation arrangement 212 is then actuated, for example, by the user drawing (e.g., a suction) on the mouthpiece or mouth-engaging end 220 of the cartridge body 200. The heating element 240 and the liquid transport element 238 are configured so as to be in a heat exchange relationship. That is, the heat generated by the heating element 240 acts to heat the aerosol precursor composition carried by the liquid transport element 238 to produce a first aerosol. The heat generated by the heating element 240 and the first aerosol are then drawn into engagement with and through the second aerosol generation arrangement 400 (i.e., through the aerosol-generating element(s) 425) toward the inhalation hole defined by the mouthpiece or mouth-engaging end 220. In some instances, the heat from the heating element 240 may interact with the aerosol-generating element(s) 425 to generate a second aerosol. The second aerosol may interact or mix with the first aerosol to form a tertiary aerosol, the tertiary aerosol being the aerosol delivered to the user by way of the mouthpiece 220 in response to the draw imparted thereto by the user. In some instances, the interaction between the heat and/or the first aerosol and the aerosol-generating element(s) 425 may cause an enhancement substance to be imparted to the first aerosol so as to produce an enhanced aerosol. For example, a medicament adsorbed on the aerosol-generating element(s) 425 may react with the first aerosol and/or the heat, or otherwise be de-adsorbed from the aerosol-generating element(s) 425 by the first aerosol and/or the heat, and combine with the first aerosol to form the enhanced aerosol. In still other instances, the aerosol-generating element(s) 425 may be configured such that interaction of the first aerosol therewith causes heat to be drawn away from the first aerosol (i.e., cooling of the first aerosol). When appropriately implemented by the user, at least the first aerosol generated by the atomizer 212 and affected by the second aerosol generation arrangement 400 aerosol are generated and drawn into the mouth of the user.

The components of the second aerosol generation arrangement 400 and/or the aerosol-generating element(s) 425 therein can vary. In general, the second aerosol generation arrangement 400 and/or the aerosol-generating element(s) 425 therein may incorporate components that can be vaporized, aerosolized or entrained in air drawn through the aerosol delivery system 100 during use. Most preferably, those components, by themselves or in cooperation with the first aerosol produced by the first aerosol generation arrangement 212, provide sensory and organoleptic effects, such as aroma, flavor, mouthfeel, visible aerosol sensations, and the like. Examples of components of the first and/or second aerosol generation arrangement 212, 400 that are drawn into the mouth of the user during draw include water (e.g., as water vapor), visible or not visible aerosol forming materials (e.g., glycerin), various volatile flavors (e.g., vanillin and menthol), volatile components of tobacco (e.g., nicotine), and the like.

A preferred aerosol-forming material produces an aerosol (whether visible or not) upon the application of sufficient heat thereto, or otherwise through the action of aerosol forming conditions using components of the aerosol delivery system. A preferred aerosol-forming material produces a visible aerosol that can be considered to be "smoke-like." A preferred aerosol-forming material is chemically simple, relative to the chemical nature of the smoke produced by burning tobacco. A preferred visible aerosol-forming material is a polyol, and exemplary preferred aerosol forming materials include glycerin, propylene glycol, and mixtures thereof. **If** desired, aerosol forming materials can be combined with other liquid materials, such as water. For example, aerosol forming material formulations can incorporate mixtures of glycerin and water, or mixtures of propylene glycol and water. See, for example, the various aerosol forming materials referenced in U.S. Pat. No. 8,678,013 to Crooks et al..

The aerosol forming materials are carried or supported by substrate materials so as to maintain those aerosol materials within the desired region of the smoking article. Exemplary substrate materials, and exemplary formulations incorporating aerosol-forming materials, are set forth in U.S. Pat. No. 4,793,365 to Sensabaugh et al.; U.S. Pat. No. 4,893,639 to White; U.S. Pat. No. 5,099,861 to Clearman et al.; U.S. Pat. No. 5,101,839 to Jakob et al.; U.S. Pat. No. 5,105,836 to Gentry et al.; U.S. Pat. No. 5,159,942 to Brinkley et al.*;* U.S. Pat. No. 5,203,355 to Clearman et al.*;* U.S. Pat. No. 5,271,419 to Arzonico et al.*;* U.S. Pat. No. 5,327,917 to Lekwauwa et al.; U.S. Pat. No. 5,396,911 to Casey, III et al.*;* U.S. Pat. No. 5,533,530 to Young et al.*;* U.S. Pat. No. 5,588,446 to Clearman; U.S. Pat. No. 5,598,868 to Jakob et al.; and U.S. Pat. No. 5,715,844 to Young et al.; and U.S. Patent Application Pub. No. 2005/0066986 to Nestor et al.. See, also, Chemical and Biological Studies on New Cigarette Prototypes that Heat Instead of Burn Tobacco, R. J. Reynolds Tobacco Company Monograph (1988). Exemplary substrate materials have been incorporated within the types of cigarettes commercially marketed under the trade names "Premier" and "Eclipse" by R. J. Reynolds Tobacco Company.

In some instances, the aerosol delivery system described with reference to FIG. 1 may be used in much the same manner as commercially marketed e-cigarettes. As a result, when smoked, a preferred aerosol delivery system 100 of the types disclosed herein may yield visible mainstream aerosol resulting principally from volatilized components of the first and second aerosol generation arrangements 212, 400, and that visible aerosol resembles in many regards the mainstream tobacco smoke of a traditional type of cigarette that bums tobacco cut filler.

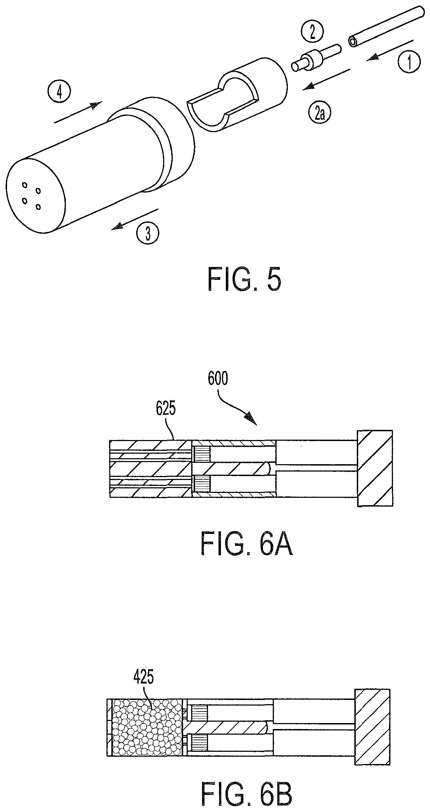

In another example, substantially the entirety of the cartridge body 200 may be formed from one or more carbon materials (see, e.g., FIG. 5), which may provide advantages over other cartridge body configurations disclosed herein in terms of biodegradability and absence of wires. In this regard, the heating element may comprise carbon foam, the reservoir may comprise carbonized fabric, and graphite may be employed to form an electrical connection with the battery and controller. Examples of a carbon-based cartridge body are provided in 2013/0255702 to Griffith et al.. In some instances, the incorporation of the second aerosol generation
arrangement disclosed herein may also be applicable to such a carbon-based cartridge body. For example, as shown in FIG. 6A and 6B, the portion 625 (see, e.g., FIG. 6A) of the cartridge element 600 disposed toward the mouthpiece of the cartridge body may be configured or otherwise alteed (see, e.g., FIG. 6B) so as to receive one or more of the aerosol-generating element(s) 425 of the types disclosed herein. In the alternative, a pre-assembled cartridge including such aerosol-generating element(s) 425 may be implemented, or the cartridge element 600 and/or the outer body receiving the cartridge element 600 may be configured to receive the first and second separating elements having the aerosol-generating elements therebetween, as otherwise disclosed herein.

Many modifications and other aspects of the disclosures set forth herein will come to mind to one skilled in the art to which these disclosures pertain having the benefit of the teachings presented in the foregoing descriptions and the associated drawings. For example, those of skill in the art will appreciate that embodiments not expressly illustrated herein may be practiced within the scope of the present disclosure, and features described herein for different embodiments may be combined with each other and/or with currently-known or future-developed technologies while remaining within the scope of the claims presented here.

## Claims

1. An aerosol delivery system (100), comprising:
a control body portion (300) including a first elongate tubular member having opposed ends, and a power source (316) disposed therein;
a cartridge body portion (200) including a second tubular member having opposed first and second ends, the first end being engaged with one of the opposed ends of the control body portion (300), the cartridge body portion (200) further comprising a first aerosol generation arrangement (212) disposed within the second tubular member and configured to operably engage the power source (316) upon
engagement between the one of the opposed ends of the control body portion (300) and the first end of the cartridge body portion (200), the second end of the cartridge body portion (200) facing toward a mouth- engaging end (220) of the aerosol delivery system (100); and
a second aerosol generation arrangement (400) disposed between the first aerosol generation arrangement (212) and the mouth-engaging end (220) of the aerosol delivery system (100), the second aerosol generation arrangement (400) being either removably engaged with the cartridge body portion (200) or housed within the second tubular member of the cartridge body portion (200), the second aerosol generation arrangement (400) comprising a cartridge having an elongate tubular plastic body and opposed end members,
wherein each of the end members are air permeable, the elongate tubular body being further configured to receive at least one aerosol-generating element and to cooperate with the opposed end members to contain the at least one aerosol-generating element therein, the cartridge being configured to be received by the second tubular member.

2. The aerosol delivery system (100) of Claim 1, wherein the second aerosol generation arrangement (400) further includes at least one aerosol- generating element (425).

3. The aerosol delivery system (100) of Claim 2, wherein the at least one aerosol-generating element (425) is selected from the group consisting of granules, pellets, beads, discrete small units, carbon pieces, extruded carbon pieces, ceramic beads, marumarized tobacco pieces, extruded or compressed cylindrical or spherical elements, milled tobacco lamina, fillers, flavors, visible aerosol forming materials, binders, ovoid elements, irregularly shaped elements, shredded pieces, flakes, elements including tobacco, elements including a visible aerosol-forming material, adsorbent objects, absorbent objects, capsules, microcapsules, a honeycomb monolith, a single porous structure, and combinations thereof.

4. The aerosol delivery system of any of Claims 1 to 3, wherein the first aerosol generation arrangement (212) comprises a liquid reservoir disposed within the second tubular member and configured to receive an aerosol precursor substance used by the first aerosol generation arrangement (212) to generate a first aerosol.

5. The aerosol delivery system (100) of Claim 4, wherein the aerosol precursor substance is one of glycerin, propylene glycol, water, saline, nicotine, and combinations thereof.

6. The aerosol delivery system (100) of Claim 5, wherein the aerosol precursor substance is one of flavorant-free and acid-free.

7. The aerosol delivery system (100) of any of Claims 1 to 6, wherein the first aerosol generation arrangement (212) includes a heating element (240) configured to provide heat for producing a first aerosol, and the second aerosol generation arrangement (400) includes at least one aerosol-generating element (425), the at least one aerosol-generating element (425) being arranged to interact with the heat and the first aerosol, drawn therethrough toward the mouth-engaging end (220), in response to a suction applied to the mouth-engaging end (220).

8. The aerosol delivery system (100) of any of Claims 1 to 7, wherein the at least one aerosol-generating element (425) of the second aerosol generation arrangement (400) is configured to interact with one of the heat from the heating element (240) of the first aerosol generation arrangement (212) and the first aerosol generated by the first aerosol generation arrangement (212) to produce a second aerosol.

9. The aerosol delivery system (100) of any of Claims 1 to 8, wherein the at least one aerosol-generating element (425) of the second aerosol- generation arrangement (400) is configured to interact with and impart an enhancement substance to the first aerosol generated by the first aerosol generation arrangement (212) to produce an enhanced aerosol, drawn toward the mouth-engaging end (220) in response to the suction applied thereto.

10. The aerosol delivery system (100) of any of Claims 1 to 9, wherein the at least one aerosol-generating element (425) of the second aerosol generation arrangement (400) is configured to interact with and remove heat from the first aerosol generated by the first aerosol generation arrangement (212) to produce a cooled aerosol, drawn toward the mouth-engaging end (220) in response to the suction applied thereto.

11. The aerosol delivery system (100) of Claims 1-10, wherein the first aerosol generated by the first aerosol generation arrangement (212) is configured to interact with the second aerosol generated by the second aerosol generation arrangement (400) to form a tertiary aerosol, drawn toward the mouth-engaging end (220) in response to the suction applied thereto.

12. The aerosol delivery system of any of Claims 1-6, wherein the first aerosol generation arrangement includes a heating element configured to provide heat for producing a first aerosol, and the second aerosol generation arrangement includes at least one aerosol-generating element, wherein the at least one aerosol-generating element of the second aerosol generation arrangement is configured to interact with one or more of the heat from the heating element of the first aerosol generation arrangement and the first aerosol generated by the first aerosol generation arrangement, drawn therethrough toward the mouth-engaging end, in response to a suction applied to the mouth-engaging end, to produce a second aerosol.

13. The aerosol delivery system of any of Claims 1-6, wherein the first aerosol generation arrangement includes a heating element configured to provide heat for producing a first aerosol, and the second aerosol generation arrangement includes at least one aerosol-generating element, wherein the at least one aerosol-generating element of the second aerosol generation arrangement is configured to interact with one of the heat from the heating element of the first aerosol generation arrangement and the first aerosol generated by the first aerosol generation arrangement, drawn therethrough toward the mouth-engaging end, in response to a suction applied to the mouth-engaging end, to produce a second aerosol.

14. The aerosol delivery system of Claim 13, wherein the at least one aerosol-generating element of the second aerosol generation arrangement is configured to interact with the first aerosol generated by the first aerosol generation arrangement to produce a second aerosol.

15. The aerosol delivery system (100) of any one of Claims 1 to 10, wherein the second aerosol generation arrangement (400) further includes a plurality of aerosol-generating elements (425) in the form of beads or pellets comprising at least one aerosol forming material.

16. The aerosol delivery system (100) of Claim 15, wherein the aerosol-generating elements (425) further comprise one or more of particulate tobacco, a tobacco extract, and nicotine, wherein the nicotine in free base form, salt form, as a complex, or as a solvate.

17. The aerosol delivery system (100) of Claim 16, wherein the aerosol-generating elements (425) further comprise one or more fillers, binders, flavorants, and combinations thereof.

18. The aerosol delivery system (100) of Claim 15, wherein the aerosol-generating elements (425) are smoke-treated.

19. A method of forming an aerosol delivery system, said method comprising:
engaging one end of a first elongate tubular member with a first end of a second tubular member, the first elongate tubular member being configured as a control body portion (300) and having a power source (316) disposed therein, and the second tubular member being configured as a cartridge body portion (200) and having a first aerosol generation arrangement (212) disposed therein, the first aerosol generation arrangement (212) being configured to operably engage the power source (316) upon engagement between the one end of the control body portion (300) and the first end of the cartridge body portion (200), the second end of the cartridge body portion (200) facing toward a mouth-engaging end (220) of the aerosol delivery system (100);
engaging a second aerosol generation arrangement (400) with the cartridge body portion (200) such that the second aerosol generation arrangement (400) is disposed between the first aerosol generation arrangement (212) and the mouth-engaging end (220) of the aerosol delivery system (100), the second aerosol generation arrangement comprising a cartridge having an elongate tubular plastic body and opposed end members, wherein each of the end members are air permeable, wherein the elongate tubular body is further configured to receive at least one aerosol-generating element and to cooperate with the opposed end members to contain the at least one aerosol-generating element therein.

20. The method of claim 19, wherein said step of engaging a second aerosol generation arrangement (400) with the cartridge body portion (200) comprises inserting the second aerosol generation arrangement (400) within the second tubular member of the cartridge body portion (200), between the first aerosol generation arrangement (212) and a second end of the second tubular member, the second end being opposed to the first end and being configured as a mouth-engaging end (220).

21. The method of Claim 20, wherein inserting the second aerosol generation arrangement (400) within the second tubular member further comprises inserting at least one aerosol-generating element (425), at least partially forming the second aerosol generation arrangement (400), into the second tubular member, the at least one aerosol-generating element (425) being selected from the group consisting of granules, pellets, beads, discrete small units, carbon pieces, extruded carbon pieces, ceramic beads, marumarized tobacco pieces, extruded or compressed cylindrical or spherical elements, milled tobacco lamina, fillers, flavors, visible aerosol forming materials, binders, ovoid elements, irregularly shaped elements, shredded pieces, flakes, elements including tobacco, elements including a visible aerosol-forming material, adsorbent objects, absorbent objects, capsules, microcapsules, a honeycomb monolith, a single porous structure, and combinations thereof.

22. The method of any of Claims 19 to 21, wherein the second aerosol generation arrangement (400) comprises a plurality of aerosol-generating elements (425) in the form of beads or pellets retained in place by a first air permeable separating element (450) disposed within the second tubular member between the first aerosol generation arrangement (212) and the second aerosol generation arrangement (400) and a second separating element (475) between the second aerosol generation arrangement (400) and the mouth-engaging end (220).

23. The method of any of Claims 19 to 22, wherein said step of engaging a second aerosol generation arrangement (400) with the cartridge body portion (200) comprises removably engaging the second aerosol generation arrangement (400) with the cartridge body portion (200), the second aerosol generation arrangement (400) comprising a first end configured to removably engage with the cartridge body portion (200) and a second end adapted to provide the mouth- engaging end (220) of the aerosol delivery system (100), and wherein the second aerosol generation arrangement (400) comprises a plurality of aerosol-generating elements (425) in the form of beads or pellets retained in place by a first air permeable separating element (450) between the first aerosol generation arrangement (212) and the second aerosol generation arrangement (400) and a second separating element (475) between the second aerosol generation arrangement (400) and the mouth-engaging end (220).

24. The method of any one of Claims 19to 23, wherein the second aerosol generation arrangement (400) comprises a plurality of aerosol-generating elements (425) in the form of beads or pellets comprising at least one aerosol forming material.

25. The method of Claim 24, wherein the aerosol-generating elements (425) further comprise one or more of particulate tobacco, a tobacco extract, and nicotine, wherein the nicotine in free base form, salt form, as a complex, or as a solvate.

26. The method of Claim 25, wherein the aerosol-generating elements (425) further comprise one or more fillers, binders, flavorants, and combinations thereof.

27. The method of Claim 26,wherein the aerosol-generating elements (425) are smoke-treated.

28. The method of any of Claims 19-27, wherein the first aerosol generation arrangement includes a heating element configured to provide heat for producing a first aerosol, and the second aerosol generation arrangement includes at least one aerosol-generating element, wherein the at least one aerosol-generating element of the second aerosol generation arrangement interacts with one of the heat from the heating element of the first aerosol generation arrangement and the first aerosol generated by the first aerosol generation arrangement, drawn therethrough toward the mouth-engaging end, in response to a suction applied to the mouth-engaging end, to produce a second aerosol.

29. The method of any of Claims 19-27, wherein the first aerosol generation arrangement includes a heating element configured to provide heat for producing a first aerosol, and the second aerosol generation arrangement includes at least one aerosol-generating element, wherein the at least one aerosol-generating element of the second aerosol generation arrangement interacts with one or more of the heat from the heating element of the first aerosol generation arrangement and the first aerosol generated by the first aerosol generation arrangement, drawn therethrough toward the mouth-engaging end, in response to a suction applied to the mouth-engaging end, to produce a second aerosol.

30. The method of Claim 28 or Claim 29, wherein the at least one aerosol-generating element of the second aerosol generation arrangement interacts with the first aerosol generated by the first aerosol generation arrangement to produce a second aerosol.

## Patentansprüche

1. Aerosolabgabesystem (100), umfassend:
einen Steuerkörperabschnitt (300), der ein erstes längliches rohrförmiges Element mit gegenüberliegenden Enden und eine darin angeordnete Energiequelle (316) beinhaltet;
einen Kartuschenkörperabschnitt (200), der ein zweites rohrförmiges Element mit gegenüberliegenden ersten und zweiten Enden beinhaltet, wobei das erste Ende mit einem der gegenüberliegenden Enden des Steuerkörperabschnitts (300) in Eingriff steht, wobei der Kartuschenkörperabschnitt (200) ferner eine erste Aerosolerzeugungsanordnung (212) umfasst, die innerhalb des zweiten rohrförmigen Elements angeordnet und konfiguriert ist, um die Energiequelle (316) funktionsfähig in Eingriff zu nehmen, beim
Eingriff zwischen dem einen der gegenüberliegenden Enden des Steuerkörperabschnitts (300) und dem ersten Ende des Kartuschenkörperabschnitts (200), wobei das zweite Ende des Kartuschenkörperabschnitts (200) zu einem mundeingreifenden Ende (220) des Aerosolabgabesystems (100) zeigt; und
eine zweite Aerosolerzeugungsanordnung (400), die zwischen der ersten Aerosolerzeugungsanordnung (212) und dem mundeingreifenden Ende (220) des Aerosolabgabesystems (100) angeordnet ist, wobei die zweite Aerosolerzeugungsanordnung (400) entweder lösbar mit dem Kartuschenkörperabschnitt (200) in Eingriff steht oder innerhalb des zweiten rohrförmigen Elements des Kartuschenkörperabschnitts (200) untergebracht ist, wobei die zweite Aerosolerzeugungsanordnung (400) eine Kartusche mit einem länglichen rohrförmigen Kunststoffkörper und gegenüberliegenden Endelementen umfasst, wobei jedes der Endelemente luftdurchlässig ist, wobei der längliche rohrförmige Körper ferner dazu ausgestaltet ist, mindestens ein Aerosolerzeugungselement aufzunehmen und mit den gegenüberliegenden Endelementen zusammenzuwirken, um das mindestens eine Aerosolerzeugungselement darin zu halten, wobei die Kartusche dazu ausgestaltet ist, von dem zweiten rohrförmigen Element aufgenommen zu werden.

2. Aerosolabgabesystem (100) nach Anspruch 1, wobei die zweite Aerosolerzeugungsanordnung (400) ferner mindestens ein Aerosolerzeugungselement (425) beinhaltet.

3. Aerosolabgabesystem (100) nach Anspruch 2, wobei das mindestens eine Aerosolerzeugungselement (425) ausgewählt ist aus der Gruppe bestehend aus Granulaten, Pellets, Perlen, diskreten kleinen Einheiten, Kohlenstoffstücken, extrudierten Kohlenstoffstücken, Keramikperlen, marumarisierten Tabakstücken, extrudierten oder komprimierten zylindrischen oder kugelförmigen Elementen, gemahlenen Tabaklamellen, Füllstoffen, Aromen, sichtbaren aerosolbildenden Materialien, Bindemitteln, eiförmigen Elementen, unregelmäßig geformten Elementen, zerkleinerten Stücken, Flocken, Elementen einschließlich Tabak, Elementen einschließlich eines sichtbaren aerosolbildenden Materials, adsorbierenden Objekten, absorbierenden Objekten, Kapseln, Mikrokapseln, einem Wabenmonolith, einer einzelnen porösen Struktur und Kombinationen davon.

4. Aerosolabgabesystem nach einem der Ansprüche 1 bis 3, wobei die erste Aerosolerzeugungsanordnung (212) ein Flüssigkeitsreservoir umfasst, das innerhalb des zweiten rohrförmigen Elements angeordnet und konfiguriert ist, um eine Aerosolvorläufersubstanz aufzunehmen, die von der ersten Aerosolerzeugungsanordnung (212) verwendet wird, um ein erstes Aerosol zu erzeugen.

5. Aerosolabgabesystem (100) nach Anspruch 4, wobei die Aerosolvorläufersubstanz eine von Glycerin, Propylenglykol, Wasser, Kochsalzlösung, Nikotin und Kombinationen davon ist.

6. Aerosolabgabesystem (100) nach Anspruch 5, wobei die Aerosolvorläufersubstanz eine von aromafreier und säurefreier ist.

7. Aerosolabgabesystem (100) nach einem der Ansprüche 1 bis 6, wobei die erste Aerosolerzeugungsanordnung (212) ein Heizelement (240) beinhaltet, das konfiguriert ist, um Wärme zur Erzeugung eines ersten Aerosols bereitzustellen, und die zweite Aerosolerzeugungsanordnung (400) mindestens ein Aerosolerzeugungselement (425) beinhaltet, wobei das mindestens eine Aerosolerzeugungselement (425) angeordnet ist, um mit der Wärme und dem ersten Aerosol zu interagieren, die durch dieses hindurch zum mundeingreifenden Ende (220) gezogen werden, als Reaktion auf ein Ansaugen, das auf das mundeingreifende Ende (220) ausgeübt wird.

8. Aerosolabgabesystem (100) nach einem der Ansprüche 1 bis 7, wobei das mindestens eine Aerosolerzeugungselement (425) der zweiten Aerosolerzeugungsanordnung (400) konfiguriert ist, um mit einem von der Wärme aus dem Heizelement (240) der ersten Aerosolerzeugungsanordnung (212) und dem ersten Aerosol, das durch die erste Aerosolerzeugungsanordnung (212) erzeugt wird, zur Erzeugung eines zweiten Aerosols zu interagieren.

9. Aerosolabgabesystem (100) nach einem der Ansprüche 1 bis 8, wobei das mindestens eine Aerosol erzeugende Element (425) der zweiten Aerosolerzeugungsanordnung (400) konfiguriert ist, um mit dem ersten Aerosol, das durch die erste Aerosolerzeugungsanordnung (212) erzeugt wird, zu interagieren und ihm eine Verstärkungssubstanz zu verleihen, um ein verstärktes Aerosol zu erzeugen, das zum mundeingreifenden Ende (220) gezogen wird, als Reaktion auf das Ansaugen, das auf das mundeingreifende Ende (220) ausgeübt wird.

10. Aerosolabgabesystem (100) nach einem der Ansprüche 1 bis 9, wobei das mindestens eine Aerosolerzeugungselement (425) der zweiten Aerosolerzeugungsanordnung (400) konfiguriert ist, um mit dem ersten Aerosol, das durch die erste Aerosolerzeugungsanordnung (212) erzeugt wird, zu interagieren und Wärme aus diesem zu entnehmen, um ein gekühltes Aerosol zu erzeugen, das zum mundeingreifenden Ende (220) gezogen wird, als Reaktion auf das Ansaugen, das auf das mundeingreifende Ende (220) ausgeübt wird.

11. Aerosolabgabesystem (100) nach Anspruch 1-10, wobei das erste Aerosol, das durch die erste Aerosolerzeugungsanordnung (212) erzeugt wird, konfiguriert ist, um mit dem zweiten Aerosol, das durch die zweite Aerosolerzeugungsanordnung (400) erzeugt wird, zu interagieren, um ein tertiäres Aerosol zu bilden, das zum mundeingreifenden Ende (220) gezogen wird, als Reaktion auf das Ansaugen, das auf das mundeingreifende Ende (220) ausgeübt wird.

12. Aerosolabgabesystem nach einem der Ansprüche 1-6, wobei die erste Aerosolerzeugungsanordnung ein Heizelement beinhaltet, das konfiguriert ist, um Wärme zur Erzeugung eines ersten Aerosols bereitzustellen, und die zweite Aerosolerzeugungsanordnung mindestens ein Aerosolerzeugungselement beinhaltet, wobei das mindestens eine Aerosolerzeugungselement der zweiten Aerosolerzeugungsanordnung konfiguriert ist, um mit einem oder mehreren von der Wärme aus dem Heizelement der ersten Aerosolerzeugungsanordnung und dem ersten Aerosol, das durch die erste Aerosolerzeugungsanordnung erzeugt wird, zu interagieren, die durch dieses hindurch zum mundeingreifenden Ende gezogen werden, als Reaktion auf ein Ansaugen, das auf das mundeingreifende Ende ausgeübt wird, um ein zweites Aerosol zu erzeugen.

13. Aerosolabgabesystem nach einem der Ansprüche 1-6, wobei die erste Aerosolerzeugungsanordnung ein Heizelement beinhaltet, das konfiguriert ist, um Wärme zur Erzeugung eines ersten Aerosols bereitzustellen, und die zweite Aerosolerzeugungsanordnung mindestens ein Aerosolerzeugungselement beinhaltet, wobei das mindestens eine Aerosolerzeugungselement der zweiten Aerosolerzeugungsanordnung konfiguriert ist, um mit einem von der Wärme aus dem Heizelement der ersten Aerosolerzeugungsanordnung und dem ersten Aerosol, das durch die erste Aerosolerzeugungsanordnung erzeugt wird, zu interagieren, die durch dieses hindurch zum mundeingreifenden Ende gezogen werden, als Reaktion auf ein Ansaugen, das auf das mundeingreifende Ende ausgeübt wird, um ein zweites Aerosol zu erzeugen.

14. Aerosolabgabesystem nach Anspruch 13, wobei das mindestens eine Aerosolerzeugungselement der zweiten Aerosolerzeugungsanordnung konfiguriert ist, um mit dem ersten Aerosol zu interagieren, das durch die erste Aerosolerzeugungsanordnung erzeugt wird, um ein zweites Aerosol zu erzeugen.

15. Aerosolabgabesystem (100) nach einem der Ansprüche 1 bis 10, wobei die zweite Aerosolerzeugungsanordnung (400) ferner eine Vielzahl von Aerosolerzeugungselementen (425) in Form von Perlen oder Pellets beinhaltet, die mindestens ein Aerosol bildendes Material umfasst.

16. Aerosolabgabesystem (100) nach Anspruch 15, wobei die Aerosolerzeugungselemente (425) ferner eines oder mehrere von teilchenförmigem Tabak, einem Tabakextrakt und Nikotin umfassen, wobei das Nikotin in Form einer freien Base, Salzform, als ein Komplex oder als ein Solvat ist.

17. Aerosolabgabesystem (100) nach Anspruch 16, wobei die Aerosolerzeugungselemente (425) ferner einen oder mehrere Füllstoffe, Bindemittel, Aromastoffe und Kombinationen derselben umfassen.

18. Aerosolabgabesystem (100) nach Anspruch 15, wobei die Aerosolerzeugungselemente (425) rauchbehandelt sind.

19. Verfahren zum Bilden eines Aerosolabgabesystems, wobei das Verfahren umfasst:
Eingreifen eines Endes eines ersten langgestreckten rohrförmigen Elements in ein erstes Ende eines zweiten rohrförmigen Elements, wobei das erste langgestreckte rohrförmige Element als Steuerkörperabschnitt (300) konfiguriert ist und eine darin angeordnete Energiequelle (316) aufweist, und das zweite rohrförmige Element als Kartuschenkörperabschnitt (200) konfiguriert ist und eine erste Aerosolerzeugungsanordnung (212) aufweist, wobei die erste Aerosolerzeugungsanordnung (212) konfiguriert ist, um die Energiequelle (316) beim Eingriff zwischen dem einen Ende des Steuerkörperabschnitts (300) und dem ersten Ende des Kartuschenkörperabschnitts (200) funktionsfähig in Eingriff zu bringen, wobei das zweite Ende des Kartuschenkörperabschnitts (200) zu einem mundeingreifenden Ende (220) des Aerosolabgabesystems (100) zeigt;
Eingreifen einer zweiten Aerosolerzeugungsanordnung (400) in den Kartuschenkörperabschnitt (200), so dass die zweite Aerosolerzeugungsanordnung (400) zwischen der ersten Aerosolerzeugungsanordnung (212) und dem mundeingreifenden Ende (220) des Aerosolabgabesystems (100) angeordnet ist, wobei das zweite Aerosolerzeugungsanordnung eine Kartusche mit einem länglichen rohrförmigen Kunststoffkörper und gegenüberliegenden Endelementen umfasst, wobei jedes der Endelemente luftdurchlässig ist, wobei der längliche rohrförmige Körper ferner dazu ausgestaltet ist, mindestens ein Aerosolerzeugungselement aufzunehmen und mit den gegenüberliegenden Endelementen zusammenzuwirken, um das mindestens eine Aerosolerzeugungselement darin zu halten.

20. Verfahren nach Anspruch 19, wobei der Schritt des Eingreifens einer zweiten Aerosolerzeugungsanordnung (400) in den Kartuschenkörperabschnitt (200) das Einsetzen der zweiten Aerosolerzeugungsanordnung (400) in das zweite rohrförmige Element des Kartuschenkörperabschnitts (200) zwischen der ersten Aerosolerzeugungsanordnung (212) und einem zweiten Ende des zweiten rohrförmigen Elements umfasst, wobei das zweite Ende dem ersten Ende gegenüberliegt und als ein mundeingreifendes Ende (220) konfiguriert ist.

21. Verfahren nach Anspruch 20, wobei das Einsetzen der zweiten Aerosolerzeugungsanordnung (400) in das zweite rohrförmige Element ferner das Einsetzen mindestens eines Aerosolerzeugungselements (425), das mindestens teilweise die zweite Aerosolerzeugungsanordnung (400) bildet, in das zweite rohrförmige Element umfasst, wobei das mindestens eine Aerosolerzeugungselement (425) ausgewählt ist aus der Gruppe bestehend aus Granulaten, Pellets, Perlen, diskreten kleinen Einheiten, Kohlenstoffstücken, extrudierten Kohlenstoffstücken, Keramikperlen, marumarisierten Tabakstücken, extrudierten oder komprimierten zylindrischen oder kugelförmigen Elemente, gemahlenen Tabaklamellen, Füllstoffen, Aromen, sichtbaren aerosolbildenden Materialien, Bindemitteln, eiförmigen Elementen, unregelmäßig geformten Elementen, zerkleinerten Stücken, Flocken, Elementen einschließlich Tabak, Elementen einschließlich eines sichtbaren aerosolbildenden Materials, adsorbierenden Objekten, absorbierenden Objekten, Kapseln, Mikrokapseln, einem Wabenmonolith, einer einzelnen porösen Struktur und Kombinationen davon.

22. Verfahren nach einem der Ansprüche 19 bis 21, wobei die zweite Aerosolerzeugungsanordnung (400) eine Vielzahl von Aerosolerzeugungselementen (425) in Form von Perlen oder Pellets umfasst, die durch ein erstes luftdurchlässiges Trennelement (450), das innerhalb des zweiten rohrförmigen Elements zwischen der ersten Aerosolerzeugungsanordnung (212) und der zweiten Aerosolerzeugungsanordnung (400) angeordnet ist, und ein zweites Trennelement (475) zwischen der zweiten Aerosolerzeugungsanordnung (400) und dem mundeingreifenden Ende (220) gehalten werden.

23. Verfahren nach einem der Ansprüche 19 bis 22, wobei der Schritt des Eingreifens einer zweiten Aerosolerzeugungsanordnung (400) in den Kartuschenkörperabschnitt (200) das lösbare Ineingriffbringen der zweiten Aerosolerzeugungsanordnung (400) mit dem Kartuschenkörperabschnitt (200) umfasst, wobei die zweite Aerosolerzeugungsanordnung (400) ein erstes Ende umfasst, das konfiguriert ist, um lösbar mit dem Kartuschenkörperabschnitt (200) in Eingriff zu kommen, und ein zweites Ende, das angepasst ist, um das mundeingreifende Ende (220) des Aerosolabgabesystems (100) bereitzustellen, und wobei die zweite Aerosolerzeugungsanordnung (400) eine Vielzahl von Aerosolerzeugungselementen (425) in Form von Perlen oder Pellets umfasst, die von einem ersten luftdurchlässigen Trennelement (450) zwischen der ersten Aerosolerzeugungsanordnung (212) und der zweiten Aerosolerzeugungsanordnung (400) und einem zweiten Trennelement (475) zwischen der zweiten Aerosolerzeugungsanordnung (400) und dem mundeingreifenden Ende (220) gehalten werden.

24. Verfahren nach einem der Ansprüche 19 bis 23, wobei die zweite Aerosolerzeugungsanordnung (400) eine Vielzahl von Aerosolerzeugungselementen (425) in Form von Perlen oder Pellets umfasst, die mindestens ein aerosolbildendes Material umfassen.

25. Verfahren nach Anspruch 24, wobei die Aerosolerzeugungselemente (425) ferner eines oder mehrere von teilchenförmigem Tabak, einem Tabakextrakt und Nikotin umfassen, wobei das Nikotin in Form einer freien Base, Salzform, als ein Komplex oder als ein Solvat ist.

26. Verfahren nach Anspruch 25, wobei die Aerosolerzeugungselemente (425) ferner einen oder mehrere Füllstoffe, Bindemittel, Aromastoffe und Kombinationen davon umfassen.

27. Verfahren nach Anspruch 26, worin die Aerosolerzeugungselemente (425) rauchbehandelt werden.

28. Verfahren nach einem der Ansprüche 19-27, wobei die erste Aerosolerzeugungsanordnung ein Heizelement beinhaltet, das konfiguriert ist, um Wärme zum Erzeugen eines ersten Aerosols bereitzustellen, und die zweite Aerosolerzeugungsanordnung mindestens ein Aerosolerzeugungselement beinhaltet, wobei das mindestens eine Aerosolerzeugungselement der zweiten Aerosolerzeugungsanordnung mit einer von der Wärme aus dem Heizelement der ersten Aerosolerzeugungsanordnung und dem ersten Aerosol, das durch die erste Aerosolerzeugungsanordnung erzeugt wird, als Reaktion auf ein auf das mundeingreifende Ende ausgeübtes Ansaugen hindurchgezogen wird, um ein zweites Aerosol zu erzeugen.

29. Verfahren nach einem der Ansprüche 19-27, wobei die erste Aerosolerzeugungsanordnung ein Heizelement beinhaltet, das konfiguriert ist, um Wärme zum Erzeugen eines ersten Aerosols bereitzustellen, und die zweite Aerosolerzeugungsanordnung mindestens ein Aerosolerzeugungselement beinhaltet, wobei das mindestens eine Aerosolerzeugungselement der zweiten Aerosolerzeugungsanordnung mit einem oder mehreren von der Wärme aus dem Heizelement der ersten Aerosolerzeugungsanordnung und dem ersten Aerosol, das durch die erste Aerosolerzeugungsanordnung erzeugt wird, interagiert, die als Reaktion auf ein auf das mundeingreifende Ende ausgeübtes Ansaugen in Richtung des mundeingreifenden Endes durchgezogen werden, um ein zweites Aerosol zu erzeugen.

30. Verfahren nach Anspruch 28 oder Anspruch 29, wobei das mindestens eine Aerosolerzeugungselement der zweiten Aerosolerzeugungsanordnung mit dem ersten Aerosol, das durch die erste Aerosolerzeugungsanordnung erzeugt wird, zusammenwirkt, um ein zweites Aerosol zu erzeugen.

## Revendications

1. Système de distribution d'aérosol (100) comprenant :
une partie de corps de commande (300) comprenant un premier organe tubulaire allongé ayant des extrémités opposées, et une source d'énergie (316) disposée à l'intérieur de cette dernière ;
une partie de corps de cartouche (200) comprenant un second organe tubulaire ayant des première et seconde extrémités opposées, la première extrémité étant mise en prise avec l'une des extrémités opposées de la partie de corps de commande (300), la partie de corps de cartouche (200) comprenant en outre un premier agencement de génération d'aérosol (212) disposé à l'intérieur du second organe tubulaire et configuré pour mettre en prise de manière opérationnelle la source d'énergie (316) suite
à la mise en prise entre l'une des extrémités opposées de la partie de corps de commande (300) et la première extrémité de la partie de corps de cartouche (200), la seconde extrémité de la partie de corps de cartouche (200) étant orientée vers une extrémité de mise en prise de bouche (220) du système de distribution d'aérosol (100) ; et
un second agencement de génération d'aérosol (400) disposé entre le premier agencement de génération d'aérosol (212) et l'extrémité de mise en prise de bouche (220) du système de distribution d'aérosol (100), le second agencement de génération d'aérosol (400) étant mis en prise de manière amovible avec la partie de corps de cartouche (200) ou logé à l'intérieur du second organe tubulaire de la partie de corps de cartouche (200), le second agencement de génération d'aérosol (400) comprenant une cartouche ayant un corps de plastique tubulaire allongé et des organes d'extrémité opposés, dans lequel chacun des organes d'extrémité sont perméables à l'air, le corps tubulaire allongé étant en outre configuré pour recevoir au moins un élément de génération d'aérosol et pour coopérer avec les organes d'extrémité opposés pour contenir l'au moins un élément de génération d'aérosol dans celui-ci, la cartouche étant configurée pour être reçu par le deuxième organe tubulaire.

2. Système de distribution d'aérosol (100) selon la revendication 1, dans lequel le second agencement de génération d'aérosol (400) comprend en outre au moins un élément de génération d'aérosol (425).

3. Système de distribution d'aérosol (100) selon la revendication 2, dans lequel l'au moins un élément de génération d'aérosol (425) est sélectionné dans le groupe comprenant les granules, les granulés, des billes, de petites unités discrètes, des pièces de carbone, des pièces de carbone extrudées, des billes en céramique, des pièces de tabac mélangées, des éléments cylindriques ou sphériques extrudés ou comprimés, des limbes de tabac broyées, des charges, des arômes, des matériaux de formation d'aérosol visibles, des liants, des éléments ovoïdes, des éléments de forme irrégulière, des pièces déchiquetées, des flocons, des éléments comprenant le tabac, des éléments comprenant un matériau de formation d'aérosol visible, des objets adsorbants, des objets absorbants, des capsules, des microcapsules, un monolithe en nid d'abeille, une structure poreuse unique, et leurs combinaisons.

4. Système de distribution d'aérosol selon l'une quelconque des revendications 1 à 3, dans lequel le premier agencement de génération d'aérosol (212) comprend un réservoir de liquide disposé à l'intérieur du second organe tubulaire et configuré pour recevoir une substance précurseur d'aérosol utilisée par le premier agencement de génération d'aérosol (212) afin de générer un premier aérosol.

5. Système de distribution d'aérosol (100) selon la revendication 4, dans lequel la substance précurseur d'aérosol est l'une parmi la glycérine, le propylène-glycol, l'eau, une solution saline, la nicotine et leurs combinaisons.

6. Système de distribution d'aérosol (100) selon la revendication 5, dans lequel la substance précurseur d'aérosol est l'une parmi une substance dépourvue d'agent aromatique et dépourvue d'acide.

7. Système de distribution d'aérosol (100) selon l'une quelconque des revendications 1 à 6, dans lequel le premier agencement de génération d'aérosol (212) comprend un élément chauffant (240) configuré pour fournir la chaleur afin de produire un premier aérosol, et le second agencement de génération d'aérosol (400) comprend au moins un élément de génération d'aérosol (425), l'au moins un élément de génération d'aérosol (425) étant agencé pour interagir avec la chaleur et le premier aérosol, aspiré à travers ce dernier vers l'extrémité de mise en prise de bouche (220), en réponse à une aspiration appliquée sur l'extrémité de mise en prise de bouche (220) .

8. Système de distribution d'aérosol (100) selon l'une quelconque des revendications 1 à 7, dans lequel l'au moins un élément de génération d'aérosol (425) du second agencement de génération d'aérosol (400) est configuré pour interagir avec l'un parmi la chaleur provenant de l'élément chauffant (240) du premier agencement de génération d'aérosol (212) et le premier aérosol généré par le premier agencement de génération d'aérosol (212) afin de produire un second aérosol.

9. Système de distribution d'aérosol (100) selon l'une quelconque des revendications 1 à 8, dans lequel l'au moins un élément de génération d'aérosol (425) du second agencement de génération d'aérosol (400) est configuré pour interagir avec et transmettre une substance d'amélioration au premier aérosol généré par le premier agencement de génération d'aérosol (212) afin de produire un aérosol amélioré, aspiré vers l'extrémité de mise en prise de bouche (220) en réponse à l'aspiration appliquée sur cette dernière.

10. Système de distribution d'aérosol (100) selon l'une quelconque des revendications 1 à 9, dans lequel l'au moins un élément de génération d'aérosol (425) du second agencement de génération d'aérosol (400) est configuré pour interagit avec et retirer la chaleur du premier aérosol généré par le premier agencement de génération d'aérosol (212) pour produire un aérosol refroidi, aspiré vers l'extrémité de mise en prise de bouche (220) en réponse à l'aspiration appliquée sur cette dernière.

11. Système de distribution d'aérosol (100) selon les revendications 1 à 10, dans lequel le premier aérosol généré par le premier agencement de génération d'aérosol (212) est configuré pour interagir avec le second aérosol généré par le second agencement de génération d'aérosol (400) pour former un aérosol tertiaire, aspiré vers l'extrémité de mise en prise de bouche (220) en réponse à l'aspiration appliquée sur cette dernière.

12. Système de distribution d'aérosol selon l'une quelconque des revendications 1 à 6, dans lequel le premier agencement de génération d'aérosol comprend un élément chauffant configuré pour fournir la chaleur pour produire le premier aérosol, et le second agencement de génération d'aérosol comprend au moins un élément de génération d'aérosol, dans lequel l'au moins un élément de génération d'aérosol du second agencement de génération d'aérosol est configuré pour interagir avec un ou plusieurs parmi la chaleur de l'élément chauffant du premier agencement de génération d'aérosol et le premier aérosol généré par le premier agencement de génération d'aérosol, aspiré à travers ce dernier vers l'extrémité de mise en prise de bouche, en réponse à une aspiration appliquée sur l'extrémité de mise en prise de bouche, afin de produire un second aérosol.

13. Système de distribution d'aérosol selon l'une quelconque des revendications 1 à 6, dans lequel le premier agencement de génération d'aérosol comprend un élément chauffant configuré pour fournir la chaleur afin de produire un premier aérosol, et le second agencement de génération d'aérosol comprend au moins un élément de génération d'aérosol, dans lequel l'au moins un élément de génération d'aérosol du second agencement de génération d'aérosol est configuré pour interagir avec l'un parmi la chaleur de l'élément chauffant du premier agencement de génération d'aérosol et le premier aérosol généré par le premier agencement de génération d'aérosol, aspiré à travers ce dernier vers l'extrémité de mise en prise de bouche, en réponse à une aspiration appliquée sur l'extrémité de mise en prise de bouche, afin de produire un second aérosol.

14. Système de distribution d'aérosol selon la revendication 13, dans lequel l'au moins un élément de génération d'aérosol du second agencement de génération d'aérosol est configuré pour interagir avec le premier aérosol généré par le premier agencement de génération d'aérosol pour produire un second aérosol.

15. Système de distribution d'aérosol (100) selon l'une quelconque des revendications 1 à 10, dans lequel le second agencement de génération d'aérosol (400) comprend en outre une pluralité d'éléments de génération d'aérosol (425) se présentant sous la forme de billes ou de granulés comprenant au moins un matériau de formation d'aérosol.

16. Système de distribution d'aérosol (100) selon la revendication 15, dans lequel les éléments de génération d'aérosol (425) comprennent en outre un ou plusieurs parmi du tabac particulaire, un extrait de tabac, et de la nicotine, dans lequel la nicotine se présente sous une forme de base libre, une forme de sel, sous forme de complexe ou sous forme de produit de solvatation.

17. Système de distribution d'aérosol (100) selon la revendication 16, dans lequel les éléments de génération d'aérosol (425) comprennent en outre un ou plusieurs parmi des charges, des liants, des arômes, et leurs combinaisons.

18. Système de distribution d'aérosol (100) selon la revendication 15, dans lequel les éléments de génération d'aérosol (425) sont traités à la fumée.

19. Procédé pour former un système de distribution d'aérosol, ledit procédé comprenant les étapes consistant à :
mettre en prise une extrémité d'un premier organe tubulaire allongé avec une première extrémité d'un second organe tubulaire, le premier organe tubulaire allongé étant configuré en tant que partie de corps de commande (300) et ayant une source d'énergie (316) disposée à l'intérieur de ce dernier, et le second organe tubulaire étant configuré en tant que partie de corps de cartouche (200) et ayant un premier agencement de génération d'aérosol (212) disposé à l'intérieur, le premier agencement de génération d'aérosol (212) étant configuré pour mettre en prise de manière opérationnelle la source d'énergie (316) suite à la mise en prise entre ladite une extrémité de la partie de corps de commande (300) et la première extrémité de la partie de corps de cartouche (200), la seconde extrémité de la partie de corps de cartouche (200) étant orientée vers une extrémité de mise en prise de bouche (220) du système de distribution d'aérosol (100) ;
mettre en prise un second agencement de génération d'aérosol (400) avec la partie de corps de cartouche (200) de sorte que le second agencement de génération d'aérosol (400) est disposé entre le premier agencement de génération d'aérosol (212) et l'extrémité de mise en prise de bouche (220) du système de distribution d'aérosol (100), le second agencement de génération d'aérosol comprenant une cartouche ayant un corps de plastique tubulaire allongé et des organes d'extrémité opposés, dans lequel chacun des organes d'extrémité est perméable à l'air, le corps tubulaire allongé étant en outre configuré pour recevoir au moins un élément de génération d'aérosol et pour coopérer avec les organes d'extrémité opposés pour contenir l'au moins un élément de génération d'aérosol dans celui-ci.

20. Procédé selon la revendication 19, dans lequel ladite étape consistant à mettre en prise un second agencement de génération d'aérosol (400) avec la partie de corps de cartouche (200) comprend l'étape consistant à insérer le second agencement de génération d'aérosol (400) à l'intérieur du second organe tubulaire de la partie de corps de cartouche (200), entre le premier agencement de génération d'aérosol (212) et une seconde extrémité du second organe tubulaire, la seconde extrémité étant opposée à la première extrémité et étant configurée en tant qu'extrémité de mise en prise de bouche (220).

21. Procédé selon la revendication 20, dans lequel l'étape consistant à insérer le second agencement de génération d'aérosol (400) à l'intérieur du second organe tubulaire comprend en outre l'étape consistant à insérer au moins un élément de génération d'aérosol (425), formant au moins partiellement le second agencement de génération d'aérosol (400), dans le second organe tubulaire, l'au moins un élément de génération d'aérosol (425) étant sélectionné dans le groupe comprenant les granules, les granulés, les billes, de petites unités discrètes, des pièces de carbone, des pièces de carbone extrudées, des billes en céramique, des pièces de tabac mélangées, des éléments cylindriques ou sphériques extrudés ou comprimés, des limbes de tabac broyées, des charges, des arômes, des matériaux de formation d'aérosol visibles, des liants, des éléments ovoïdes, des éléments de forme irrégulière, des pièces déchiquetées, des flocons, des éléments comprenant du tabac, des éléments comprenant un matériau de formation d'aérosol visible, des objets adsorbants, des objets absorbants, des capsules, des microcapsules, un monolithe en nid d'abeille, une structure poreuse unique et leurs combinaisons.

22. Procédé selon l'une quelconque des revendications 19 à 21, dans lequel le second agencement de génération d'aérosol (400) comprend une pluralité d'éléments de génération d'aérosol (425) se présentant sous forme de billes ou de granulés retenus en place par un premier élément de séparation perméable à l'air (450) disposé à l'intérieur du second organe tubulaire entre le premier agencement de génération d'aérosol (212) et le second agencement de génération d'aérosol (400) et un second élément de séparation (475) entre le second agencement de génération d'aérosol (400) et l'extrémité de mise en prise de bouche (220) .

23. Procédé selon l'une quelconque des revendications 19 à 22, dans lequel ladite étape consistant à mettre en prise un second agencement de génération d'aérosol (400) avec la partie de corps de cartouche (200) comprend l'étape consistant à mettre en prise, de manière amovible, le second agencement de génération d'aérosol (400) avec la partie de corps de cartouche (200), le second agencement de génération d'aérosol (400) comprenant une première extrémité configurée pour se mettre en prise, de manière amovible, avec la partie de corps de cartouche (200) et une seconde extrémité adaptée pour fournir l'extrémité de mise en prise de bouche (220) du système de distribution d'aérosol (100), et dans lequel le second agencement de génération d'aérosol (400) comprend une pluralité d'éléments de génération d'aérosol (425) se présentant sous la forme de billes ou de granulés retenus en place par un premier élément de séparation perméable à l'air (450) entre le premier agencement de génération d'aérosol (212) et le second agencement de génération d'aérosol (400) et un second élément de séparation (475) entre le second agencement de génération d'aérosol (400) et l'extrémité de mise en prise de bouche (220).

24. Procédé selon l'une quelconque des revendications 19 à 23, dans lequel le second agencement de génération d'aérosol (400) comprend une pluralité d'éléments de génération d'aérosol (425) se présentant sous la forme de billes ou de granulés comprenant au moins un matériau de formation d'aérosol.

25. Procédé selon la revendication 24, dans lequel les éléments de génération d'aérosol (425) comprennent en outre un ou plusieurs parmi du tabac particulaire, un extrait de tabac, et de la nicotine, dans lequel la nicotine se présente sous une forme de base libre, sous forme de sel, sous forme de complexe ou sous forme de produit de solvatation.

26. Procédé selon la revendication 25, dans lequel les éléments de génération d'aérosol (425) comprennent en outre un ou plusieurs parmi des charges, des liants, des arômes et leurs combinaisons.

27. Procédé selon la revendication 26, dans lequel les éléments de génération d'aérosol (425) sont traités à la fumée.

28. Procédé selon l'une quelconque des revendications 19 à 27, dans lequel le premier agencement de génération d'aérosol comprend un élément chauffant configuré pour fournir de la chaleur afin de produire un premier aérosol, et le second agencement de génération d'aérosol comprend au moins un élément de génération d'aérosol, dans lequel l'au moins un élément de génération d'aérosol du second agencement de génération d'aérosol interagit avec l'un parmi la chaleur de l'élément chauffant du premier agencement de génération d'aérosol et le premier aérosol généré par le premier agencement de génération d'aérosol, aspiré à travers ce dernier vers l'extrémité de mise en prise de bouche, en réponse à une aspiration appliquée sur l'extrémité de mise en prise de bouche, pour produire un second aérosol.

29. Procédé selon l'une quelconque des revendications 19 à 27, dans lequel le premier agencement de génération d'aérosol comprend un élément chauffant configuré pour fournir la chaleur afin de produire un premier aérosol, et le second agencement de génération d'aérosol comprend au moins un élément de génération d'aérosol, dans lequel l'au moins un élément de génération d'aérosol du second agencement de génération d'aérosol interagit avec un ou plusieurs parmi la chaleur de l'élément chauffant du premier agencement de génération d'aérosol et le premier aérosol généré par le premier agencement de génération d'aérosol, aspiré à travers ce dernier vers l'extrémité de mise en prise de bouche, en réponse à une aspiration appliquée sur l'extrémité de mise en prise de bouche, afin de produire un second aérosol.

30. Procédé selon la revendication 28 ou la revendication 29, dans lequel l'au moins un élément de génération d'aérosol du second agencement de génération d'aérosol interagit avec le premier aérosol généré par le premier agencement de génération d'aérosol pour produire un second aérosol.
